(19) Europäisches Patentamt
European Patent Office
Office européen des brevets

(11) **EP 3 981 864 A1**

(12) **EUROPEAN PATENT APPLICATION**

(43) Date of publication:
**13.04.2022 Bulletin 2022/15**

(21) Application number: **20200964.3**

(22) Date of filing: **09.10.2020**

(51) International Patent Classification (IPC):
***C11D 1/83*** *(2006.01)* ***C11D 1/94*** *(2006.01)*
***C11D 3/386*** *(2006.01)* ***C11D 11/00*** *(2006.01)*
***C12N 9/04*** *(2006.01)* ***C12N 9/02*** *(2006.01)*
***C12N 9/88*** *(2006.01)*

(52) Cooperative Patent Classification (CPC):
**C12N 9/0071; C11D 1/83; C11D 1/94; C11D 3/2075; C11D 3/33; C11D 3/361; C11D 3/38654; C11D 3/38663; C11D 11/0023; C12N 9/0006; C12N 9/0069; C12N 9/88;**
C12Y 114/99

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **The Procter & Gamble Company
Cincinnati, OH 45202 (US)**

(72) Inventors:
- **GONZALES, Denis Alfred
1853 Strombeek-Bever (BE)**
- **RUPARD, Spencer Christopher
Cincinnati, Ohio 45202 (US)**
- **VELASQUEZ, Juan Esteban
Cincinnati, Ohio 45202 (US)**

(74) Representative: **P&G Patent Belgium UK
N.V. Procter & Gamble Services Company S.A.
Temselaan 100
1853 Strombeek-Bever (BE)**

Remarks:
The complete document including Reference Tables and the Sequence Listing can be downloaded from the EPO website

(54) **DETERGENT COMPOSITION**

(57) A manual dishwashing detergent composition having fatty acid alpha-dioxygenases and methods of using said compositions to provide a benefit by converting long chain fatty acids present in soils into 2-hydroperoxy fatty acids or terminal aldehydes.

EP 3 981 864 A1

Printed by Jouve, 75001 PARIS (FR)

## Description

REFERENCE TO A SEQUENCE LISTING

This application contains a Sequence Listing in computer readable form. The computer readable form is incorporated herein by reference.

FIELD OF THE INVENTION

The present invention relates to enzyme-containing detergent compositions which provide one or more benefits, including good cleaning particularly good grease emulsification, and long-lasting suds especially in the presence of greasy soils.

BACKGROUND OF THE INVENTION

Detergent compositions should have a good suds profile in particular a long-lasting suds profile especially in the presence of greasy soils while providing good soil and/or grease cleaning. Users usually see suds as an indicator of the performance of the detergent composition. Moreover, the user of a detergent composition may also use the suds profile and the appearance of the suds (e.g., density, whiteness) as an indicator that the wash solution still contains active detergent ingredients. This is particularly the case for manual washing, also referred to herein as hand-washing, where the user usually doses the detergent composition depending on the suds remaining and renews the wash solution when the suds subsides or when the suds does not look thick enough. Thus, a detergent composition, particularly a manual wash detergent composition that generates little or low density suds would tend to be replaced by the user more frequently than is necessary. Accordingly, it is desirable for a detergent composition to provide "good sudsing profile", which includes good suds height and/or density as well as good suds duration during the initial mixing of the detergent with water and/or during the entire washing operation.

The need also exists for an improved detergent composition, when used in a manual-washing process, the composition preferably also provides a pleasant washing experience, *i.e,* good feel on the user's hands during the wash. Preferably detergent compositions are also easy to rinse. Further it is desirous that the improved detergent composition is stable and will not phase separate, resulting in greater shelf-life of the product. Preferably in addition, the composition provides a good finish to the washed items. There is also the desire to reduce the amount of surfactants without negatively impacting sudsing nor grease cleaning and emulsification profile. Thus, there is the need to find new compositions that improve cleaning and suds longevity in hand washing conditions.

It has been found that some types of soil, in particular greasy soils comprising fatty acids, act as a suds suppressor, triggering consumers to replace the product more frequently than is necessary.

Hydroperoxy fatty acid producing enzymes have been found to alleviate the suds suppression from the presence of greasy soils comprising fatty acids. However, a need remains for a detergent compositions with further improved suds properties in the presence of greasy soils comprising fatty acids, and that at the same time while providing good soil and grease removal.

US 2017/321161 A1 relates to the use of a detergent composition comprising a fatty acid-transforming enzyme to impart suds longevity in a washing process, as well as a method for promoting suds longevity in a washing process for washing soiled articles, comprising the step of: delivering a composition comprising a fatty acid-transforming enzyme to a volume of water to form a wash liquor and immersing the soiled article in the liquor. US 2003/166485 A1 relates to catalytically bleaching substrates, especially laundry fabrics, with a bleaching catalyst in the presence of an enzymatic bleach enhancing system. "Water Soluble Film Flakes Incorporating Functional Ingredients", IP.COM JOURNAL, IP.COM INC., WEST HENRIETTA, NY, US,2 January 2014, relates generally to water-soluble compositions useful for delivering functional ingredients, including enzymes. More particularly, the disclosure relates to particularly defined water soluble film flakes containing one or more functional ingredients, and related compositions containing such flakes, wherein the related compositions can be powders, liquids, or gels, for example, and wherein the combinations may be used, for example, as detergents. EP3483243 A1 relates to a detergent composition, preferably a manual dishwashing detergent composition, comprising: one or more hydroperoxy fatty acid producing enzymes; one or more hydroperoxy fatty acid converting enzymes; and a surfactant system, as well as a method of washing comprising the detergent composition. EP3483251 A1 relates to a liquid detergent composition, preferably a liquid manual dishwashing detergent composition, comprising one or more hydroperoxy fatty acid producing enzymes selected from the group consisting of: arachidonate lipoxygenases, alpha-dioxygenases, and mixtures thereof, preferably alpha-dioxygenases, a surfactant system and a liquid carrier (i.e., water), and methods of washing comprising the liquid detergent composition. EP3483244 A1 relates to a detergent composition, preferably a manual dishwashing detergent composition, comprising a surfactant system and at least one fatty acid processing fusion enzyme, wherein the at least one fatty acid processing fusion enzyme

comprises at least two catalytic domains: i.e. a hydroperoxy fatty acid producing domain and a hydroperoxy fatty acid converting domain, and methods of using such compositions.

SUMMARY OF THE INVENTION

The present invention, as described in claim 1, provides a detergent composition with desirable suds properties, even in the presence of greasy soils comprising fatty acids, while at the same time providing good soil and grease removal.
The detergent composition is particularly suited for manually washing soiled articles, preferably dishware, using the method described in claim 14.
The elements of the composition of the invention described in relation to the first aspect of the invention apply *mutatis mutandis* to the other aspects of the invention.

BRIEF DESCRIPTION OF THE DRAWINGS

While the specification concludes with claims particularly pointing out and distinctly claiming the subject matter that is regarded as the present disclosure, it is believed that the disclosure will be more fully understood from the following description taken in conjunction with the accompanying drawings.

FIG. 1 Illustration of Sequence Similarity.

DETAILED DESCRIPTION OF THE INVENTION

The liquid hand dishwashing cleaning compositions of the present invention provide a good sudsing profile, including high suds volume generation and sustained suds stabilization through the dishwashing process, even when in presence of greasy. This signals to the user that there remains sufficient active ingredients present to provide continued cleaning performance, as such triggering less re-dosing and overconsumption of the product by the user.
The compositions of the present invention also provide good grease removal, in particular good removal of uncooked grease and particulate soils.

Definitions

As used herein, articles such as "a" and "an" when used in a claim, are understood to mean one or more of what is claimed or described.
The term "comprising" as used herein means that steps and ingredients other than those specifically mentioned can be added. This term encompasses the terms "consisting of" and "consisting essentially of." The compositions of the present invention can comprise, consist of, and consist essentially of the essential elements and limitations of the invention described herein, as well as any of the additional or optional ingredients, components, steps, or limitations described herein.
The term "dishware" as used herein includes cookware and tableware made from, by non-limiting examples, ceramic, china, metal, glass, plastic (e.g., polyethylene, polypropylene, polystyrene, etc.) and wood.
The term "grease" or "greasy" as used herein means materials comprising at least in part (*i.e.,* at least 0.5 wt% by weight of the grease) saturated and unsaturated fats and oils, preferably oils and fats derived from animal sources such as beef, pig and/or chicken.
The terms "include", "includes" and "including" are meant to be non-limiting.
The term "particulate soils" as used herein means inorganic and especially organic, solid soil particles, especially food particles, such as for non-limiting examples: finely divided elemental carbon, baked grease particle, and meat particles.
The term "sudsing profile" as used herein refers to the properties of a cleaning composition relating to suds character during the dishwashing process. The term "sudsing profile" of a cleaning composition includes suds volume generated upon dissolving and agitation, typically manual agitation, of the cleaning composition in the aqueous washing solution, and the retention of the suds during the dishwashing process. Preferably, hand dishwashing cleaning compositions characterized as having "good sudsing profile" tend to have high suds volume and/or sustained suds volume, particularly during a substantial portion of or for the entire manual dishwashing process. This is important as the consumer uses high suds as an indicator that sufficient cleaning composition has been dosed. Moreover, the consumer also uses the sustained suds volume as an indicator that sufficient active cleaning ingredients (e.g., surfactants) are present, even towards the end of the dishwashing process. The consumer usually renews the washing solution when the sudsing subsides. Thus, a low sudsing cleaning composition will tend to be replaced by the consumer more frequently than is necessary because of the low sudsing level.

It is understood that the test methods that are disclosed in the Test Methods Section of the present application must be used to determine the respective values of the parameters of Applicants' inventions as described and claimed herein.

In all embodiments of the present invention, all percentages are by weight of the total composition, as evident by the context, unless specifically stated otherwise. All ratios are weight ratios, unless specifically stated otherwise, and all measurements are made at 25°C, unless otherwise designated.

As used herein the term "fragment" means an amino acid sequence of at least 30, 60, 100, 150 contiguous amino acids of the reference sequences or any integer there between.

As used herein the term "identity" means the identity between two or more sequences and is expressed in terms of the identity or similarity between the sequences as calculated over the entire length of a sequence aligned against the entire length of the reference sequence. Sequence identity can be measured in terms of percentage identity; the higher the percentage, the more identical the sequences are. The percentage identity is calculated over the length of comparison. For example, the identity is typically calculated over the entire length of a sequence aligned against the entire length of the reference sequence. Methods of alignment of sequences for comparison are well known in the art and identity can be calculated by many known methods. Various programs and alignment algorithms are described in the art. It should be noted that the terms 'sequence identity' and 'sequence similarity' can be used interchangeably.

As used herein the term "increased suds longevity" means an increase in the duration of visible suds in a washing process cleaning soiled articles using the composition comprising one or more of the hydroperoxy fatty acid producing enzymes described herein compared with the suds longevity provided by the same composition and process in the absence of the hydroperoxy fatty acid producing enzymes, or comprising alternative hydroperoxy fatty acid producing enzymes.

Key targeted soiled surfaces by this application are soiled dishware.

As used herein, the term "variant" of hydroperoxy fatty acid producing enzyme such as "variant" of fatty acid alpha-dioxygenase enzyme according to the invention means an amino acid sequence when the hydroperoxy fatty acid producing enzyme is modified by, or at, one or more amino acids (for example 1, 2, 3, 4, 5, 6, 7, 8, 9 or 10 or more amino acid modifications) selected from substitutions, insertions, deletions and combinations thereof. The variant may have "conservative" substitutions, wherein a substituted amino acid has similar structural or chemical properties to the amino acid that replaces it, for example, replacement of leucine with isoleucine. A variant may have "non-conservative" changes, for example, replacement of a glycine with a tryptophan. Variants may also include sequences with amino acid deletions or insertions, or both. Guidance in determining which amino acid residues may be substituted, inserted, or deleted without abolishing the activity of the protein may be found using computer programs well known in the art. Variants may also include truncated forms derived from a wild-type hydroperoxy fatty acid producing enzyme, such as for example, a protein with a truncated N-terminus. Variants may also include forms derived by adding an extra amino acid sequence to a wild-type protein, such as for example, an N-terminal tag, a C-terminal tag or an insertion in the middle of the protein sequence.

As used herein, the term "water hardness" or "hardness" means uncomplexed cation ions (*i.e.,* $Ca^{2+}$ or $Mg^{2+}$) present in water that have the potential to precipitate with anionic surfactants or any other anionically charged detergent actives under alkaline conditions, and thereby diminishing the surfactancy and cleaning capacity of surfactants. Further, the terms "high water hardness" and "elevated water hardness" can be used interchangeably and are relative terms for the purposes of the present invention, and are intended to include, but not limited to, a hardness level containing at least 12 grams of calcium ion per gallon water (gpg, "American grain hardness" units).

## Detergent Composition

A preferred detergent composition is a manual dishwashing composition, preferably in liquid form. It typically contains from 30% to 95%, preferably from 40% to 90%, more preferably from 50% to 85% by weight of the composition of a liquid carrier in which the other essential and optional components are dissolved, dispersed or suspended. One preferred component of the liquid carrier is water.

Preferably the pH of the detergent composition of the invention, measured as a 10% product concentration in demineralized water at 20°C, is adjusted to between 3 and 14, more preferably between 4 and 13, more preferably between 6 and 12 and most preferably between 8 and 10. The pH of the detergent composition can be adjusted using pH modifying ingredients known in the art.

The composition of the present invention can be Newtonian or non-Newtonian, preferably Newtonian. Preferably, the composition has a viscosity of from 50 mPa·s to 5,000 mPa·s, more preferably from 300 mPa·s to 2,000 mPa·s, or most preferably from 500 mPa·s to 1,500 mPa·s, alternatively combinations thereof. The viscosity is measured at 20°C with a Brookfield RT Viscometer using spindle 31 with the RPM of the viscometer adjusted to achieve a torque of between 40% and 60%.

Enzymes

Fatty acids can be oxidized in the presence of molecular oxygen ($O_2$) by oleate dioxygenases, linoleate lipoxygenases, arachidonate lipoxygenases, and alpha-dioxygenases, to produce hydroperoxy fatty acids. These hydroperoxylated compounds can be further processed by other enzymes or spontaneously transform to a diverse group of oxygenated fatty acids and other derivatives. In the context of the current application, a "hydroperoxy fatty acid producing enzyme" is an enzyme that is capable of converting at least one fatty acid into a mixture of oxygenated compounds, comprising at least a hydroperoxy fatty acid as an intermediate or as a final product.

Unexpectedly, the Applicants found that a group of fatty acid alpha-dioxygenase enzymes are capable of producing a more stable hence longer lasting sudsing profile in hand dishwashing detergent wash solutions comprising oily and/or greasy soils. Not wishing to be bound by theory, the Applicants believe that the increased sudsing benefits are due to the improved conversion of fatty acids, present in the oily and/or greasy soils, into fatty acids derivatives with enhanced surfactant properties and/or decreased tendency to precipitation in the presence of hard water.

Accordingly, the detergent composition of the invention comprises one or more hydroperoxy fatty acid producing enzymes selected from fatty acid alpha-dioxygenases.

Fatty acids can be oxidized in the presence of molecular oxygen ($O_2$) by fatty acid alpha-dioxygenases (αDOX), which convert saturated and unsaturated fatty acids to their corresponding 2-hydroperoxy fatty acids *via* stereoselective oxygenation at the alpha carbon. The resulting 2-hydroperoxy fatty acids can undergo spontaneous decarboxylation to shorter aldehydes or can be reduced to 2-hydroxy fatty acids in the presence of a reducing agent. αDOX are generally encoded by different species of plants and fungi, where they are up-regulated during the host defense response against pathogen attack, but homologs are also found in bacteria. Alpha-dioxygenases are grouped under the InterPRO family IPR034815 and members of such family are included as part of the current invention.

Crystal structures of *Oryza sativa* αDOX (SEQ ID NO: 1; PDB ID: 4KVJ, 4KVK, and 4KVL) and *Arabidopsis thaliana* αDOX (SEQ ID NO: 3; PDB ID: 4HHR, and 4HHS) have been published, revealing the substrate binding site and the active site residues. These structures, together with sequence alignments, suggest that several amino acids may contribute to and/or define the active site of the enzyme. For instance, in SEQ ID NO: 1, the residues H311, Y379, H382 and R559, which are highly conserved in the family, are important for catalysis. Residues N145, (H/R)157, W213, D214, S216, Y219, G220, R230, G256, G264, H276, N277, A308, K309, H311, W315, F375, Y379, R380, H382, S435, G437, N448, Y520, G532, F549, F552, R559, and G579 are also highly conserved, but their roles in catalysis have not been well established.

The fatty acid alpha-dioxygenase can comprise a polypeptide sequence comprising the amino acids H311, Y379, H382 and R559, wherein said positions are numbered with reference to SEQ ID NO: 1. In embodiments of the present invention, the fatty acid alpha-dioxygenase comprises a polypeptide sequence comprising the amino acids N145, (H/R)157, W213, D214, S216, Y219, G220, R230, G256, G264, H276, N277, A308, K309, H311, W315, F375, Y379, R380, H382, S435, G437, N448, Y520, G532, F549, F552, R559, and G579, wherein said positions are numbered with reference to SEQ ID NO: 1.

The fatty acid alpha-dioxygenase can comprise a polypeptide sequence comprising one or more sequence motifs selected from the group consisting of: FGRN, N-$(X)_2$-T-X-WWD-X-S, WD-X-S-$(X)_2$-YG, F-$(X)_2$-EHN-$(X)_2$-CD, ANW-X-G, AK-X-H, YR-X-H, PYS-X-TE-X-F-$(X)_2$-VYR-X-H-X-L, R-X-RER-X-V-X-RYN-X-FRR, MA-X-RRL-$(X)_2$-DRF, and combinations thereof; wherein X represents any amino acid. In embodiments, the fatty acid alpha-dioxygenase comprises a polypeptide sequence comprising the sequence motifs: FGRN, N-$(X)_2$-T-X-WWD-X-S, WD-X-S-$(X)_2$-YG, F-$(X)_2$-EHN-$(X)_2$-CD, ANW-X-G, AK-X-H, YR-X-H, PYS-X-TE-X-F-$(X)_2$-VYR-X-H-X-L, R-X-RER-X-V-X-RYN-X-FRR, and MA-X-RRL-$(X)_2$-DRF; wherein X represents any amino acid.

The alpha-dioxygenase can comprise a polypeptide sequence selected from the group consisting of: SEQ ID NO: 276, 277, and 278; preferably SEQ ID NO: 276, and 277; and most preferably SEQ ID NO: 276. In the referred to polypeptide sequences, the X in the SEQ IDs are chosen such that polypeptide sequence selected from the group consisting of: SEQ ID NO: 276, 277, and 278 have at least 65% identity to one or more sequences selected from the group consisting of: SEQ ID NO: 1, 2, 3, 4, 5, 6, 7, and functional fragments thereof. More preferably, the X in the SEQ IDs are chosen such that polypeptide sequence selected from the group consisting of: SEQ ID NO: 276, 277, and 278 have at least at least 70%, preferably at least 80%, preferably at least 90%, preferably at least 95%, preferably at least 98% or preferably even 100% identity to one or more sequences selected from the group consisting of: SEQ ID NO: 1, 2, and their functional fragments; more preferably SEQ ID NO: 1.

Not wishing to be bound by theory, in several alpha-dioxygenases, the pro-R hydrogen covalently bound to carbon 2 of a fatty acid is removed utilizing a tyrosyl radical, followed by addition of molecular oxygen to generate a 2-hydroperoxy fatty acid. In *Oryza sativa* αDOX (SEQ ID NO 1), such tyrosyl radical is generated from Tyr379. Alternatively, a cysteine thiyl radical could catalyze such reaction. In embodiments of the present invention, the fatty acid alpha-dioxygenase comprises a polypeptide sequence comprising a tyrosine or a cysteine at position 379, wherein said position is numbered with reference to SEQ ID NO: 1. A suitable example of an alpha-dioxygenase comprising a cysteine at

position 379 is SEQ ID NO 140; wherein said position is numbered with reference to SEQ ID NO: 1.

The cleaning composition comprises fatty acid alpha-dioxygenases. Preferred alpha-dioxygenases exhibit at least 65%, preferably at least 70%, preferably at least 80%, preferably at least 90%, preferably at least 95%, preferably at least 98% or preferably even 100% identity as calculated over the entire length of a sequence aligned against the entire length of at least one reference sequence selected from the group consisting of wild-type alpha-dioxygenases selected from the group consisting of: SEQ ID NO: 1, 2, 3, 4, 5, 6, 7, and their functional fragments thereof.

Preferably the fatty acid alpha-dioxygenases are present in an amount of from 0.0001 wt% to 1 wt%, by weight of the composition, based on active protein in the composition. More preferably the fatty acid alpha-dioxygenases are present in the amounts of from 0.001 wt% to 0.2 wt%, by weight of the composition, based on active protein in the composition.

The fatty acids being converted by the fatty acid alpha-dioxygenases can be selected from the group consisting of: mono unsaturated fatty acids, di unsaturated fatty acids, tri unsaturated fatty acids, tetra unsaturated fatty acids, penta unsaturated fatty acids, hexa unsaturated fatty acids, saturated fatty acids, and mixtures thereof; preferably myristoleic acid, myristic acid, pentadecanoic acid, palmitoleic acid, palmitic acid, sapienic acid, margaric acid, oleic acid, elaidic acid, vaccenic acid, linoleic acid, linoelaidic acid, $\alpha$-linolenic acid, $\gamma$-linolenic acid, stearic acid, gadoleic acid, arachidic acid, behenic acid, $\alpha$-eleostearic acid, $\beta$-eleostearic acid, ricinoleic acid, eicosenic acid, arachidonic acid, eicosapentaenoic acid, erucic acid, docosadienoic acid, docosahexaenoic acid, tetracosenoic acid, and mixtures thereof, preferably palmitic acid, stearic acid, oleic acid, and linoleic acid, and mixtures thereof.

The cleaning composition can comprises a fatty acid alpha-dioxygenase; having at least 65%, at least about 70%, at least about 80% identity to one or more sequences selected from the group consisting of: SEQ ID NO: 1, 2, 3, 4, 5, 6, 7, and their functional fragments thereof; preferably SEQ ID NO: 1, 2, and their functional fragments; and most preferably SEQ ID NO: 1, and its functional fragments. Suitable fatty acid alpha-dioxygenases with at least about 65% identity to SEQ ID NO: 1 are SEQ ID NO: 24, and 26. Suitable fatty acid alpha-dioxygenases with at least about 80% identity to SEQ ID NO: 1 are SEQ ID NO: 15, 16, 17, 18, 19, 20, 21, 22, 23, 25, 28, 29, 30, 31, 32, 33, 34, 35, 36, 37, 38, 39, 40, 41, 42, 44, 46, 47, 48, 49, 50, 51, 52, 53, 54, 55, 56, 57, and 58. Suitable fatty acid alpha-dioxygenases with at least about 65% identity to SEQ ID NO: 2 are SEQ ID NO: 60, 61, and 68. Suitable fatty acid alpha-dioxygenases with at least about 70% identity to SEQ ID NO: 2 are SEQ ID NO: 59, 62, 63, 64, 65, 66, 67, 69, and 70.

The cleaning composition can comprise a fatty acid alpha-dioxygenase, wherein said alpha-dioxygenase comprises a polypeptide sequence having at least about 90%, 95%, 98%, 100% identity to one or more sequences selected from the group consisting of: SEQ ID NO: 1, 2, 3, 4, 5, 6, 7, 15, 16, 17, 18, 19, 20, 21, 22, 23, 24, 25, 26, 28, 29, 30, 31, 32, 33, 34, 35, 36, 37, 38, 39, 40, 41, 42, 44, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 82, 83, 84, 85, 86, 87, 89, 90, 91, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, and their functional fragments thereof; preferably SEQ ID NO: 1, 2, 15, 16, 17, 18, 19, 20, 21, 22, 23, 24, 25, 26, 28, 29, 30, 31, 32, 33, 34, 35, 36, 37, 38, 39, 40, 41, 42, 44, 46, 47, 48, 49, 50, 51, 52, 53, 54, 55, 56, 57, 58, 59, 60, 61, 62, 63, 64, 65, 66, 67, 68, 69, 70, and their functional fragments thereof; more preferably SEQ ID NO: 1, 15, 16, 17, 18, 19, 20, 21, 22, 23, 24, 25, 26, 28, 29, 30, 31, 32, 33, 34, 35, 36, 37, 38, 39, 40, 41, 42, 44, 46, 47, 48, 49, 50, 51, 52, 53, 54, 55, 56, 57, 58; most preferably SEQ ID NO: 1.

Identity, or homology, percentages as mentioned herein in respect of the present invention are those that can be calculated, for example, with AlignX obtainable from Thermo Fischer Scientific or with the alignment tool from Uniprot (https://www.uniprot.org/align/). Alternatively, a manual alignment can be performed. For enzyme sequence comparison the following settings can be used: Alignment algorithm: Needleman and Wunsch, J. Mol. Biol. 1970, 48: 443-453. As a comparison matrix for amino acid similarity the Blosum62 matrix is used (Henikoff S. and Henikoff J.G., P.N.A.S. USA 1992, 89: 10915-10919). The following gap scoring parameters are used: Gap opening penalty: -10, gap extension penalty: -1.

A given sequence is typically compared against the full-length sequence or fragments of SEQ ID NO: 1, 2, 3, 4, 5, 6, 7, 15, 16, 17, 18, 19, 20, 21, 22, 23, 24, 25, 26, 28, 29, 30, 31, 32, 33, 34, 35, 36, 37, 38, 39, 40, 41, 42, 44, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 82, 83, 84, 85, 86, 87, 89, 90, 91, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206,

207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, or 247 to obtain a score. Polypeptides of the present disclosure include polypeptides containing an amino acid sequence having at least 65%, at least about 70%, at least about 75%, at least about 80%, at least about 85%, at least about 90%, at least about 95%, at least about 98%, at least about 99%, or 100% identity to the amino acid sequence of any one of SEQ ID NO: 1, 2, 3, 4, 5, 6, 7, 15, 16, 17, 18, 19, 20, 21, 22, 23, 24, 25, 26, 28, 29, 30, 31, 32, 33, 34, 35, 36, 37, 38, 39, 40, 41, 42, 44, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 82, 83, 84, 85, 86, 87, 89, 90, 91, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, and 247. Suitable polypeptides also include polypeptides having at least about 10, at least about 12, at least about 14, at least about 16, at least about 18, at least about 20, at least about 30, at least about 40, at least about 50, at least about 60, at least about 70, or at least about 80 consecutive amino acids of the amino acid sequence of any one of SEQ ID NO: 1, 2, 3, 4, 5, 6, 7, 15, 16, 17, 18, 19, 20, 21, 22, 23, 24, 25, 26, 28, 29, 30, 31, 32, 33, 34, 35, 36, 37, 38, 39, 40, 41, 42, 44, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 82, 83, 84, 85, 86, 87, 89, 90, 91, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, and 247.

The present invention also includes compositions comprising variants of fatty acid alpha-dioxygenases, as previously described. Variants of fatty acid alpha-dioxygenases include polypeptide sequences resulting from modification of a wild-type fatty acid alpha-dioxygenase at one or more amino acids. A variant includes a "modified enzyme" or a "mutant enzyme" which encompasses proteins having at least one substitution, insertion, and/or deletion of an amino acid. A modified enzyme may have 1, 2, 3, 4, 5, 6, 7, 8, 9 or 10 or more amino acid modifications (selected from substitutions, insertions, deletions and combinations thereof).

The variants may have "conservative" substitutions. Suitable examples of conservative substitution includes one conservative substitution in the enzyme, such as a conservative substitution in SEQ ID NO: 1, 2, 3, 4, 5, 6, 7, 15, 16, 17, 18, 19, 20, 21, 22, 23, 24, 25, 26, 28, 29, 30, 31, 32, 33, 34, 35, 36, 37, 38, 39, 40, 41, 42, 44, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 82, 83, 84, 85, 86, 87, 89, 90, 91, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, and their functional fragments thereof. Other suitable examples include 10 or fewer conservative substitutions in the protein, such as five or fewer. An enzyme of the invention may therefore include 1, 2, 3, 4, 5, 6, 7, 8, 9, 10 or more conservative substitutions. An enzyme can be produced to contain one or more conservative substitutions by manipulating the nucleotide sequence that encodes that enzyme using, for example, standard procedures such as site-directed mutagenesis or PCR. Examples of amino acids which may be substituted for an original amino acid in an enzyme and which are regarded as conservative substitutions include: Ser for Ala; Lys for Arg; Gln or His for Asn; Glu for Asp; Asn for Gln; Asp for Glu; Pro for Gly; Asn or Gln for His; Leu or Val for Ile; Ile or Val for Leu; Arg or Gln for Lys; Leu or Ile for Met; Met, Leu or Tyr for Phe; Thr for Ser; Ser for Thr; Tyr for Trp; Trp or Phe for Tyr; and Ile or Leu for Val.

It is important that variants of enzymes retain and preferably improve the ability of the wild-type protein to catalyze the conversion of the fatty acids. Some performance drop in a given property of variants may of course be tolerated, but the variants should retain and preferably improve suitable properties for the relevant application for which they are intended. Screening of variants of one of the wild-types can be used to identify whether they retain and preferably improve appropriate properties.

The alpha-dioxygenase polypeptides described herein are not restricted to the genetically encoded amino acids. Thus, in addition to the genetically encoded amino acids, the polypeptides described herein may be comprised,

either in whole or in part, of naturally-occurring and/or synthetic non-encoded amino acids. Certain commonly encountered non-encoded amino acids of which the polypeptides described herein may be comprised include, but are not limited to: the D-stereoisomers of the genetically-encoded amino acids; 2,3-diaminopropionic acid (Dpr); $\alpha$-aminoisobutyric acid (Aib); $\varepsilon$-aminohexanoic acid (Aha); $\delta$-aminovaleric acid (Ava); N-methylglycine or sarcosine (MeGly or Sar); ornithine (Orn); citrulline (Cit); t-butylalanine (Bua); t-butylglycine (Bug); N-methylisoleucine (Melle); phenylglycine (Phg); cyclohexylalanine (Cha); norleucine (Nle); naphthylalanine (Nal); 2-chlorophenylalanine (Oct); 3-chlorophenylalanine (Mcf); 4-chlorophenylalanine (Pcf); 2-fluorophenylalanine (Off); 3-fluorophenylalanine (Mff); 4-fluorophenylalanine (Pff); 2-bromophenylalanine (Obf); 3-bromophenylalanine (Mbf); 4-bromophenylalanine (Pbf); 2-methylphenylalanine (Omf); 3-methylphenylalanine (Mmf); 4-methylphenylalanine (Pmf); 2-nitrophenylalanine (Onf); 3-nitrophenylalanine (Mnf); 4-nitrophenylalanine (Pnf); 2-cyanophenylalanine (Ocf); 3-cyanophenylalanine (Mcf); 4-cyanophenylalanine (Pcf); 2-trifluoromethylphenylalanine (Otf); 3-trifluoromethylphenylalanine (Mtf); 4-trifluoromethylphenylalanine (Ptf); 4-aminophenylalanine (Paf); 4-iodophenylalanine (Pif); 4-aminomethylphenylalanine (Pamf); 2,4-dichlorophenylalanine (Opcf); 3,4-dichlorophenylalanine (Mpcf); 2,4-difluorophenylalanine (Opff); 3,4-difluorophenylalanine (Mpff); pyrid-2-ylalanine (2pAla); pyrid-3-ylalanine (3pAla); pyrid-4-ylalanine (4pAla); naphth-1-ylalanine (1nAla); naphth-2-ylalanine (2nAla); thiazolylalanine (taAla); benzothienylalanine (bAla); thienylalanine (tAla); furylalanine (fAla); homophenylalanine (hPhe); homotyrosine (hTyr); homotryptophan (hTrp); pentafluorophenylalanine (5ff); styrylkalanine (sAla); authrylalanine (aAla); 3,3-diphenylalanine (Dfa); 3-amino-5-phenylpentanoic acid (Afp); penicillamine (Pen); 1,2,3,4-tetrahydroisoquinoline-3-carboxylic acid (Tic); $\beta$-2-thienylalanine (Thi); methionine sulfoxide (Mso); N(w)-nitroarginine (nArg); homolysine (hLys); phosphonomethylphenylalanine (pmPhe); phosphoserine (pSer); phosphothreonine (pThr); homoaspartic acid (hAsp); homoglutamic acid (hGlu); 1-aminocyclopent-(2 or 3)-ene-4 carboxylic acid; pipecolic acid (PA), azetidine-3-carboxylic acid (ACA); 1-aminocyclopentane-3-carboxylic acid; allylglycine (aOly); propargylglycine (pgGly); homoalanine (hAla); norvaline (nVal); homoleucine (hLeu), homovaline (hVal); homoisoleucine (hIle); homoarginine (hArg); N-acetyl lysine (AcLys); 2,4-diaminobutyric acid (Dbu); 2,3-diaminobutyric acid (Dab); N-methylvaline (MeVal); homocysteine (hCys); homoserine (hSer); hydroxyproline (Hyp) and homoproline (hPro). Additional non-encoded amino acids of which the polypeptides described herein may be comprised will be apparent to those of skill in the art. These amino acids may be in either the L- or D-configuration.

The composition can also include variants in the form of truncated forms or fragments derived from a wild-type enzyme, such as a protein with a truncated N-terminus or a truncated C-terminus. The composition can comprise variants of alpha-dioxygenase enzymes that comprise a fragment of any of the alpha-dioxygenase polypeptides described herein that retain the functional alpha-dioxygenase activity and/or an improved property of an engineered alpha-dioxygenase polypeptide. Accordingly, the composition can comprise a polypeptide fragment having alpha-dioxygenase activity (e.g., capable of converting substrate to product under suitable reaction conditions), wherein the fragment comprises at least about 80%, 90%, 95%, 98%, or 99% of a full-length amino acid sequence of an engineered polypeptide of the present invention. Some enzymes may include an N-terminal signal peptide that is likely removed upon secretion by the cell. The composition can comprise variants without the N-terminal signal peptide. Bioinformatic tools, such as SignalP ver 4.1 (Petersen TN., Brunak S., von Heijne G. and Nielsen H. (2011), Nature Methods, 8:785-786), can be used to predict the existence and length of such signal peptides.

The composition can comprise an alpha-dioxygenase enzyme having an amino acid sequence comprising an insertion as compared to any one of the alpha-dioxygenase polypeptide sequences described herein. The insertions can comprise one or more amino acids, 2 or more amino acids, 3 or more amino acids, 4 or more amino acids, 5 or more amino acids, 6 or more amino acids, 8 or more amino acids, 10 or more amino acids, 15 or more amino acids, or 20 or more amino acids, where the associated functional activity and/or improved properties of the alpha-dioxygenase described herein is maintained. The insertions can be to amino or carboxy terminus, or internal portions of the alpha-dioxygenase polypeptide. The composition can comprise variants derived by adding an extra amino acid sequence, such as an N-terminal tag or a C-terminal tag. Non-limiting examples of tags are maltose binding protein (MBP) tag, glutathione S-transferase (GST) tag, thioredoxin (Trx) tag, His-tag, and any other tags known by those skilled in art. Tags can be used to improve solubility and expression levels during fermentation or as a handle for enzyme purification.

Enzymes can also be modified by a variety of chemical techniques to produce derivatives having essentially the same or preferably improved activity as the unmodified enzymes, and optionally having other desirable properties. For example, carboxylic acid groups of the protein, whether carboxyl-terminal or side chain, may be provided in the form of a salt of a pharmaceutically-acceptable cation or esterified, for example to form a C1-C6 alkyl ester, or converted to an amide, for example of formula CONR1R2 wherein R1 and R2 are each independently H or C1-C6 alkyl, or combined to form a heterocyclic ring, such as a 5- or 6-membered ring. Amino groups of the enzyme, whether amino-terminal or side chain, may be in the form of a pharmaceutically-acceptable acid addition salt, such as the HCl, HBr, acetic acid, benzoic acid, toluene sulfonic acid, maleic acid, tartaric acid and other organic acid addition salts, or may be modified to C1-C20 alkyl or dialkyl amino or further converted to an amide. Hydroxyl groups of the protein side chains may be converted to alkoxy or ester groups, for example C1-C20 alkoxy or C1-C20 alkyl ester, using well-recognized techniques. Phenyl and phenolic rings of the protein side chains may be substituted with one or more halogen atoms, such as F, Cl,

Br or I, or with C1-C20 alkyl, C1-C20 alkoxy, carboxylic acids and esters thereof, or amides of such carboxylic acids. Methylene groups of the protein side chains can be extended to homologous C2-C4 alkylenes. Thiols can be protected with any one of a number of well-recognized protecting groups, such as acetamide groups. Those skilled in the art will also recognize methods for introducing cyclic structures into the proteins of this disclosure to select and provide conformational constraints to the structure that result in enhanced stability.

The enzymes can be provided on a solid support, such as a membrane, resin, solid carrier, or other solid phase material. A solid support can be composed of organic polymers such as polystyrene, polyethylene, polypropylene, polyfluoroethylene, polyethyleneoxy, and polyacrylamide, as well as co-polymers and grafts thereof. A solid support can also be inorganic, such as glass, silica, controlled pore glass (CPG), reverse phase silica or metal, such as gold or platinum. The configuration of a solid support can be in the form of beads, spheres, particles, granules, a gel, a membrane or a surface. Surfaces can be planar, substantially planar, or non-planar. Solid supports can be porous or non-porous, and can have swelling or non-swelling characteristics. A solid support can be configured in the form of a well, depression, or other container, vessel, feature, or location.

The polypeptides can have alpha-dioxygenase activity that is bound or immobilized on the solid support such that they retain at least a portion of their improved properties relative to a reference polypeptide (e.g., SEQ ID NO: 1). Accordingly, it is further contemplated that any of the methods of using the alpha-dioxygenase polypeptides can be carried out using the same alpha-dioxygenase polypeptides bound or immobilized on a solid support.

The alpha-dioxygenase polypeptide can be bound non-covalently or covalently. Various methods for conjugation and immobilization of enzymes to solid supports (e.g., resins, membranes, beads, glass, etc.) are well known in the art. Other methods for conjugation and immobilization of enzymes to solid supports (e.g., resins, membranes, beads, glass, etc.) are well known in the art (See, e.g., Yi et al., Proc. Biochem., 42: 895-898 [2007]; Martin et al., Appl. Microbiol. Biotechnol., 76: 843-851 [2007]; Koszelewski et al. J. Mol. Cat. B: Enz., 63: 39-44 [2010]; Truppo et al., Org. Proc. Res. Develop., published online: dx.doi.org/10.1021/op200157c; and Mateo et al., Biotechnol. Prog., 18:629-34 [2002], etc.). Solid supports useful for immobilizing the alpha-dioxygenase polypeptides of the present invention include, but are not limited to, beads or resins comprising polymethacrylate with epoxide functional groups, polymethacrylate with amino epoxide functional groups, styrene/DVB copolymer or polymethacrylate with octadecyl functional groups.

The enzymes may be incorporated into the cleaning compositions *via* an additive particle, such as an enzyme granule or in the form of an encapsulate or may be added in the form of a liquid formulation. Encapsulating the enzymes promote the stability of the enzymes in the composition and helps to counteract the effect of any hostile compounds present in the composition, such as bleach, protease, surfactant, chelant, etc. The fatty acid alpha-dioxygenase enzymes may be the only enzymes in the additive particle or may be present in the additive particle in combination with one or more additional co-enzymes.

The cleaning composition can comprise the fatty acid alpha-dioxygenase, wherein said fatty acid alpha-dioxygenase is present in an amount of from 0.0001 wt% to 1 wt%, preferably from 0.001 wt% to 0.2 wt%, by weight of the cleaning composition, based on active protein.

The cleaning composition can further comprise one or more co-enzymes selected from the group consisting of: fatty-acid peroxidases (EC 1.11.1.3), unspecific peroxygenases (EC 1.11.2.1), plant seed peroxygenases (EC 1.11.2.3), fatty acid peroxygenases (EC1.11.2.4), linoleate diol synthases (EC 1.13.11.44), 5,8-linoleate diol synthases (EC 1.13.11.60 and EC 5.4.4.5), 7,8-linoleate diol synthases (EC 1.13.11.60 and EC 5.4.4.6), 9,14-linoleate diol synthases (EC 1.13.11.B1), 8,11-linoleate diol synthases, oleate diol synthases, other linoleate diol synthases, unspecific monooxygenase (EC 1.14.14.1), alkane 1-monooxygenase (EC 1.14.15.3), oleate 12-hydroxylases (EC 1.14.18.4), fatty acid amide hydrolases (EC 3.5.1.99), fatty acid photoalpha-dioxygenases (EC 4.1.1.106), oleate hydratases (EC 4.2.1.53), linoleate isomerases (EC 5.2.1.5), linoleate (10E,12Z)-isomerases (EC 5.3.3.B2), non-heme fatty acid decarboxylases, P450 fatty acid decarboxylases, amylases, lipases, proteases, cellulases, and mixtures thereof; preferably fatty-acid peroxidases (EC 1.11.1.3), unspecific peroxygenases (EC 1.11.2.1), plant seed peroxygenases (EC 1.11.2.3), and fatty acid peroxygenases (EC1.11.2.4), non-heme fatty acid decarboxylases, P450 fatty acid decarboxylases, and mixtures thereof.

Where necessary, the composition comprises, provides access to, or forms *in situ* any additional substrate necessary for the effective functioning of the enzyme. For example, molecular oxygen can be provided as an additional substrate for fatty acid alpha-dioxygenases. Molecular oxygen can be obtained from the atmosphere or from a precursor that can be transformed to produce oxygen in situ. In many applications, oxygen from the atmosphere can be present in sufficient amounts. In embodiments, the cleaning composition may be supplemented with heme and/or a source of iron to enhance or facilitate the conversion of the fatty acids.

The fatty acid alpha-dioxygenase can comprise a heme cofactor selected from the group comprising: heme a, heme b, heme c, heme d, heme i, heme m, heme o, heme s, their derivatives, and mixtures thereof; preferably heme b. Alternatively, the heme cofactor is covalently attached to the fatty acid alpha-dioxygenases.

The fatty acid alpha-dioxygenase can comprise a heme cofactor comprising: a) a porphyrin group and b) a metal. Non-limiting examples of porphyrin groups are: protoporphyrin IX, N-methyl protoporphyrin IX, protoporphyrin IX

monomethyl ester, protoporphyrin IX dimethyl ester, protoporphyrin IX diamide, protoporphyrin IX bis thiosulfate, porphin, phthalocyanine, octaethylporphoyrin, tetraphenylporphyrin, and their derivatives; preferably protoporphyrin IX. Non-limiting examples of metals are: Mg, Al, V, Cr, Mn, Fe, Co, Ni, Cu, Zn, Ga, Ge, Mo, Tc, Ru, Rh, Pd, Ag, Cd, In, Sn, Sb, W, Re, Os, Ir, Pt, Au, Hg, Tl, Pb, Bi, and mixtures thereof; preferably Fe. The fatty acid alpha-dioxygenase can comprise a heme cofactor comprising a cation selected from the group consisting of: $Mg^{2+}$, $Cr^{3+}$, $Mn^{3+}$, $Fe^{3+}$, $Co^{3+}$, $Ni^{2+}$, $Cu^{2+}$, $Zn^{2+}$, $Ga^{3+}$, $Rh^{2+}$, $Pd^{2+}$, $Ag^{2+}$, $In^{3+}$, $Sn^{4+}$, $VO^{2+}$, and mixtures thereof; preferably $Fe^{3+}$. The fatty acid alpha-dioxygenase can comprise a heme cofactor comprising an axially bound ligand. Examples of ligands are: chloride, methyl group, carbonyl group, hydroxide group, and tetrahydrofuran.

## Surfactant System

Preferably the detergent composition comprises from 1% to 60%, preferably from 5% to 50%, more preferably from 8% to 40%, by weight of the total composition of a surfactant system.

The surfactant system of the composition of the present invention preferably comprises one or more anionic surfactants. Preferably, the surfactant system for the detergent composition of the present invention comprises from 1% to 40%, preferably 6% to 35%, more preferably 8% to 30% by weight of the total composition of the anionic surfactants. The anionic surfactants can be any anionic cleaning surfactants, preferably selected from sulfate and/or sulfonate anionic surfactants. HLAS (linear alkylbenzene sulfonates) would be the most preferred sulfonate anionic surfactants. Especially preferred anionic surfactants are selected from the group consisting of alkyl sulfates, alkyl alkoxy sufates and mixtures thereof, and preferably wherein the alkyl alkoxy sulfates are alkyl ethoxy sulfates. Preferred anionic surfactants are a combination of alkyl sulfates and alkyl ethoxy sulfates with a combined average ethoxylation degree of less than 5, preferably less than 3, more preferably less than 2 and more than 0.5 and an average level of branching of from about 5% to about 40%, more preferably from about 10% to 40%, and even more preferably from about 20% to 40%.

The alkyl sulfate anionic surfactant has an alkyl chain comprising an average of from 8 to 18 carbon atoms, preferably from 10 to 14 carbon atoms, more preferably from 12 to 13 carbon atoms.

The alkyl chain of the alkyl sulfated anionic surfactant preferably has a mol fraction of C12 and C13 chains of at least 50%, preferably at least 65%, more preferably at least 80%, most preferably at least 90%. Suds mileage is particularly improved, especially in the presence of greasy soils, when the C13/C12 mol ratio of the alkyl chain is at least 50/50, preferably from 60/40 to 80/20, most preferably from 60/40 to 70/30, while not compromising suds mileage in the presence of particulate soils.

The average alkoxylation degree is the mol average alkoxylation degree of all the components of the mixture (*i.e.,* mol average alkoxylation degree) of the anionic surfactants. In the mol average alkoxylation degree calculation the weight of sulfate anionic surfactant components not having alkoxylate groups should also be included.

$$\text{Mol average alkoxylation degree} = (x1 * \text{alkoxylation degree of surfactant } 1 + x2 * \text{alkoxylation degree of surfactant } 2 + \ldots) / (x1 + x2 + \ldots)$$

wherein x1, x2, ... are the number of moles of each sulfate anionic surfactant of the mixture and alkoxylation degree is the number of alkoxy groups in each sulfate anionic surfactant.

The average level of branching is the weight average % of branching and it is defined according to the following formula:

$$\text{Weight average of branching (\%)} = [(x1 * \text{wt\% branched alcohol 1 in alcohol } 1 + x2 * \text{wt\% branched alcohol 2 in alcohol } 2 + \ldots) / (x1 + x2 + \ldots)] * 100$$

wherein x1, x2, ... are the weight in grams of each alcohol in the total alcohol mixture of the alcohols which were used as starting material for the anionic surfactant for the composition of the invention. In the weight average branching degree calculation the weight of anionic surfactant components not having branched groups should also be included.

Suitable counterions for the anionic surfactant include alkali metal cation earth alkali metal cation, alkanolammonium or ammonium or substituted ammonium, but preferably sodium.

Suitable examples of commercially available sulfates include, those based on Neodol alcohols ex the Shell company, Lial - Isalchem and Safol ex the Sasol company, natural alcohols ex The Procter & Gamble Chemicals company. Suitable sulfonate surfactants for use herein include water-soluble salts of C8-C18 alkyl or hydroxyalkyl sulfonates; C11-C18 alkyl benzene sulfonates (LAS), modified alkylbenzene sulfonate (MLAS); methyl ester sulfonate

(MES); and alpha-olefin sulfonate (AOS). Those also include the paraffin sulfonates may be monosulfonates and/or disulfonates, obtained by sulfonating paraffins of 10 to 20 carbon atoms. The sulfonate surfactants also include the alkyl glyceryl sulfonate surfactants.

If ethoxylated alkyl sulfate is present, without wishing to be bound by theory, through tight control of processing conditions and feedstock material compositions, both during alkoxylation especially ethoxylation and sulfation steps, the amount of 1,4-dioxane by-product within alkoxylated especially ethoxylated alkyl sulfates can be reduced. Based on recent advances in technology, a further reduction of 1,4-dioxane by-product can be achieved by subsequent stripping, distillation, evaporation, centrifugation, microwave irradiation, molecular sieving or catalytic or enzymatic degradation steps. Processes to control 1,4-dioxane content within alkoxylated/ethoxylated alkyl sulfates have been described extensively in the art. Alternatively 1,4-dioxane level control within detergent formulations has also been described in the art through addition of 1,4-dioxane inhibitors to 1,4-dioxane comprising formulations, such as 5,6-dihydro-3-(4-morpholinyl)-1-[4-(2-oxo-1-piperidinyl)-phenyl]-2-(1-H)-pyridone, 3-$\alpha$-hydroxy-7-oxo stereoisomer-mixtures of cholinic acid, 3-(N-methyl amino)-L-alanine, and mixtures thereof.

The surfactant system of the composition of the present invention preferably comprises a primary co-surfactant system, wherein the primary co-surfactant system is preferably selected from the group consisting of amphoteric surfactant, zwitterionic surfactant and mixtures thereof. Preferably the amphoteric surfactant is an amine oxide surfactant and the zwitterionic surfactant is a betaine surfactant. Preferably, the surfactant system for the detergent composition of the present invention comprises from 0.5% to 15%, preferably from 1% to 12%, more preferably from 2% to 10%, by weight of the total composition of a primary co-surfactant system.

Preferably the weight ratio of the anionic surfactants to the primary co-surfactants is less than 9:1, more preferably from 5:1 to 1:1, more preferably from 4:1 to 2:1. Preferably the primary co-surfactant system is an amphoteric surfactant. Preferably, the primary co-surfactant system is an amine oxide surfactant, and wherein the composition comprises anionic surfactant and amine oxide surfactant in a weight ratio of less than 9:1, more preferably from 5:1 to 1:1, more preferably from 4:1 to 2:1, preferably from 3:1 to 2.5:1. Preferably the composition of the present invention, wherein the surfactant system comprises one or more anionic surfactants and one or more co-surfactants, wherein the anionic surfactants are a mixture of alkyl sulfates and alkyl alkoxy sulfates, the co-surfactants are alkyl dimethyl amine oxides, and wherein the weight ratio of the anionic surfactants to the co-surfactants is from 4:1 to 2:1.

Preferred amine oxides are alkyl dimethyl amine oxide or alkyl amido propyl dimethyl amine oxide, more preferably alkyl dimethyl amine oxide and especially coco dimethyl amino oxide, especially coconut oil derived C12-14 alkyl dimethyl amine oxide. Amine oxide may have a linear or branched alkyl moiety, preferably a linear alkyl moiety.

The composition may further comprise a C10AO, especially n-decyl dimethyl amine, and preferably comprises less than 5% preferably less than 3% by weight of total amine oxide of a C8 amine oxide such as a C8 dimethyl amine oxide.

Preferably the primary co-surfactant system is a zwitterionic surfactant. Suitable exampes of zwitterionic surfactants include betaines, preferably alkyl betaines, alkylamidobetaines, and mixtures thereof. Cocoamidopropylbetaine is most preferred.

Preferably, the surfactant system of the composition of the present invention further comprises from 0.1% to 10% by weight of the total composition of a secondary co-surfactant system preferably comprising a non-ionic surfactant. Suitable non-ionic surfactants include the condensation products of aliphatic alcohols with on average from 1 to 25 moles of ethylene oxide. The alkyl chain of the aliphatic alcohol can either be straight or branched, primary or secondary, and generally contains on average from 8 to 22 carbon atoms. Particularly preferred are the condensation products of alcohols having an alkyl group containing on average from 8 to 18 carbon atoms, preferably from 8 to 15 carbon atoms with on average from 2 to 18 moles, preferably 2 to 15 moles, more preferably 5-12 moles of ethylene oxide per mole of alcohol. Highly preferred non-ionic surfactants are the condensation products of guerbet alcohols with on average from 2 to 18 moles, preferably 2 to 15 moles, more preferably 5-12 moles of ethylene oxide per mole of alcohol. Preferably, the non-ionic surfactants are an alkyl ethoxylated surfactants, preferably comprising on average from 9 to 15 carbon atoms in its alkyl chain and on average from 5 to 12 units of ethylene oxide per mole of alcohol. Other suitable non-ionic surfactants for use herein include fatty alcohol polyglycol ethers, alkylpolyglucosides and fatty acid glucamides, preferably alkylpolyglucosides.

Alkyl polyglucoside nonionic surfactants are typically more sudsing than other nonionic surfactants such as alkyl ethoxylated alcohols. In addition, they have been found to improve suds mileage in the presence of particulate soils.

A combination of alkyl sulfate anionic surfactant and alkylpolyglucoside nonionic surfactant has been found to also improve polymerized grease removal, suds mileage performance, reduced viscosity variation with changes in the surfactant and/or surfactant system, and a more sustained Newtonian rheology.

The alkyl polyglucoside surfactant can be selected from C6-C18 alkyl polyglucoside surfactant. The alkyl polyglucoside surfactant can have a number average degree of polymerization of from 0.1 to 3.0, preferably from 1.0 to 2.0, more preferably from 1.2 to 1.6. The alkyl polyglucoside surfactant can comprise a blend of short chain alkyl polyglucoside surfactant having an alkyl chain comprising 10 carbon atoms or less, and mid to long chain alkyl polyglucoside surfactant having an alkyl chain comprising greater than 10 carbon atoms to 18 carbon atoms, preferably from

12 to 14 carbon atoms.

Short chain alkyl polyglucoside surfactants have a monomodal chain length distribution between C8-C10, mid to long chain alkyl polyglucoside surfactants have a monomodal chain length distribution between C10-C18, while mid chain alkyl polyglucoside surfactants have a monomodal chain length distribution between C12-C14. In contrast, C8 to C18 alkyl polyglucoside surfactants typically have a monomodal distribution of alkyl chains between C8 and C18, as with C8 to C16 and the like. As such, a combination of short chain alkyl polyglucoside surfactants with mid to long chain or mid chain alkyl polyglucoside surfactants have a broader distribution of chain lengths, or even a bimodal distribution, than non-blended C8 to C18 alkyl polyglucoside surfactants. Preferably, the weight ratio of short chain alkyl polyglucoside surfactant to long chain alkyl polyglucoside surfactant is from 1:1 to 10:1, preferably from 1.5:1 to 5:1, more preferably from 2:1 to 4:1. It has been found that a blend of such short chain alkyl polyglucoside surfactant and long chain alkyl polyglucoside surfactant results in faster dissolution of the detergent solution in water and improved initial sudsing, in combination with improved suds stability.

The anionic surfactant and alkyl polyglucoside surfactant can be present at a weight ratio of from greater than 1:1 to 10:1, preferably from 1.5:1 to 5:1, more preferably from 2:1 to 4:1

C8-C16 alkyl polyglucosides are commercially available from several suppliers (e.g., Simusol® surfactants from Seppic Corporation; and Glucopon® 600 CSUP, Glucopon® 650 EC, Glucopon® 600 CSUP/MB, and Glucopon® 650 EC/MB, from BASF Corporation). Glucopon® 215UP is a preferred short chain APG surfactant. Glucopon® 600CSUP is a preferred mid to long chain APG surfactant.

In preferred compositions, the surfactant system can comprise an alkyl sulfate anionic surfactant having an average degree of branching of less than 15% and alkyl polyglucoside nonionic surfactant.

Enzyme Stabilizer

Preferably the composition of the invention further comprises an enzyme stabilizer, selected from the group consisting of chemical and physical stabilizers, preferably the physical stabilizer comprises encapsulating the enzyme. Suitable enzyme stabilizers may be selected from the group consisting of (a) univalent, bivalent and/or trivalent cations preferably selected from the group of inorganic or organic salts of alkaline earth metals, alkali metals, aluminum, iron, copper and zinc, preferably alkali metals and alkaline earth metals, preferably alkali metal and alkaline earth metal salts with halides, sulfates, sulfites, carbonates, hydrogencarbonates, nitrates, nitrites, phosphates, formates, acetates, propionates, citrates, maleates, tartrates, succinates, oxalates, lactates, and mixtures thereof. In a preferred embodiment the salt is selected from the group consisting of sodium chloride, calcium chloride, potassium chloride, sodium sulfate, potassium sulfate, sodium acetate, potassium acetate, sodium formate, potassium formate, calcium lactate, calcium nitrate and mixtures thereof. Most preferred are salts selected from the group consisting of calcium chloride, potassium chloride, potassium sulfate, sodium acetate, potassium acetate, sodium formate, potassium formate, calcium lactate, calcium nitrate, and mixtures thereof, and in particular potassium salts selected from the group of potassium chloride, potassium sulfate, potassium acetate, potassium formate, potassium propionate, potassium lactate and mixtures thereof. Most preferred are potassium acetate and potassium chloride. Preferred calcium salts are calcium formate, calcium lactate and calcium nitrate including calcium nitrate tetrahydrate. Calcium and sodium formate salts may be preferred. These cations are present at at least 0.01 wt%, preferably at least 0.03 wt%, more preferably at least 0.05 wt%, most preferably at least 0.25 wt% up to 2 wt% or even up to 1 wt% by weight of the total composition. These salts are formulated from 0.1 wt% to 5 wt%, preferably from 0.2 wt% to 4 wt%, more preferably from 0.3 wt% to 3 wt%, most preferably from 0.5 wt% to 2 wt% relative to the total weight of the composition. Further enzyme stabilizers can be selected from the group (b) carbohydrates selected from the group consisting of oligosaccharides, polysaccharides and mixtures thereof, such as a monosaccharide glycerate as described in WO201219844; (c) mass efficient reversible protease inhibitors selected from the group consisting of phenyl boronic acid and derivatives thereof, preferably 4-formyl phenylboronic acid; (d) alcohols such as 1,2-propane diol, propylene glycol; (e) peptide aldehyde stabilizers such as tripeptide aldehydes such as Cbz-Gly-Ala-Tyr-H, or disubstituted alaninamide; (f) carboxylic acids such as phenyl alkyl dicarboxylic acid as described in WO2012/19849 or multiply substituted benzyl carboxylic acid comprising a carboxyl group on at least two carbon atoms of the benzyl radical such as described in WO2012/19848, phthaloyl glutamine acid, phthaloyl asparagine acid, aminophthalic acid and/or an oligoamino-biphenyl-oligocarboxylic acid; and (g) mixtures thereof.

Additional Enzymes

Preferred compositions of the invention comprise one or more additional enzymes selected from the group consisting of amylases, lipases, proteases, cellulases, lipoxygenases, diol synthases, and mixtures thereof. Even more preferred compositions of the invention comprise one or more enzymes selected from lipases, proteases, cellulases, amylases and any combination thereof. Most preferably compositions of the invention comprise one or more enzymes selected from lipases, proteases, amylases and any combination thereof.

It may be particularly preferred for the compositions of the present invention to additionally comprise a protease enzyme. Since oleic acid and other foam suppressing unsaturated fatty acids are present in body soils or even human skin, as protease enzyme acts as a skin care agent, or breaks down proteinaceous soils, fatty acids released are broken down, preventing suds suppression.

It may be particularly preferred for the compositions of the present invention to additionally comprise an amylase enzyme. Since oily soils are commonly entrapped in starchy soils, the amylase and unsaturated fatty acid transforming enzymes work synergistically together: fatty acid soils are released by breakdown of starchy soils with amylase, thus, the unsaturated fatty acid transforming enzyme is particularly effective in ensuring there is no negative impact on suds in the wash liquor.

Each additional enzyme is typically present in an amount of from 0.0001 wt% to 1 wt%, preferably from 0.0005 wt% to 0.5 wt%, more preferably from 0.005 wt% to 0.1 wt%, by weight of the composition, based on active protein.

Salt

The composition of the present invention may optionally comprise from 0.01% to 3%, preferably from 0.05% to 2%, more preferably from 0.2% to 1.5%, or most preferably 0.5% to 1%, by weight of the total composition of a salt, preferably a monovalent, divalent inorganic salt or a mixture thereof, preferably sodium chloride. Most preferably the composition alternatively or further comprises a multivalent metal cation in the amount of from 0.01 wt% to 2 wt%, preferably from 0.1% to 1%, more preferably from 0.2% to 0.8% by weight of the composition, preferably the multivalent metal cation is magnesium, aluminum, copper, calcium or iron, more preferably magnesium, most preferably said multivalent salt is magnesium chloride. Without wishing to be bound by theory, it is believed that use of a multivalent cation helps with the formation of protein/ protein, surfactant/ surfactant or hybrid protein/ surfactant network at the oil water and air water interface that is strengthening the suds.

Carbohydrates

Preferably the composition of the present invention comprises one or more carbohydrates selected from the group comprising O-glycan, N-glycan, and mixtures thereof. Suitable carbohydrates include alpha or beta glucan with 1,3 and/or 1.4 and/or 1,6 linkage. Glucans can be modified especially with carboxyl sulfate, glycol ether of amino groups. Glucan can be extracted from dextran, starch or cellulose. Glucan with structure close to natural glucan such as schizophyllan, scleroglucan or paramylon are particularly preferred.

Hydrotrope

The composition of the present invention may optionally comprise from 1% to 10%, or preferably from 0.5% to 10%, more preferably from 1% to 6%, or most preferably from 0.1% to 3%, or combinations thereof, by weight of the total composition of a hydrotrope, preferably sodium cumene sulfonate. Other suitable hydrotropes for use herein include anionic-type hydrotropes, particularly sodium, potassium, and ammonium xylene sulfonate, sodium, potassium and ammonium toluene sulfonate, sodium potassium and ammonium cumene sulfonate, and mixtures thereof, as disclosed in U.S. Patent 3,915,903.

Organic Solvent

The composition of the present invention may optionally comprise an organic solvent. Preferably the organic solvents include alcohols, glycols, and glycol ethers, alternatively alcohols and glycols. The composition comprises from 0% to less than 50%, preferably from 0.01% to 25%, more preferably from 0.1% to 10%, or most preferably from 0.5% to 5%, by weight of the total composition of an organic solvent, preferably an alcohol, more preferably an ethanol, a polyalkyleneglycol, more preferably polypropyleneglycol, and mixtures thereof.

Amphiphilic Polymer

The composition of the present invention may further comprise from about 0.01% to about 5%, preferably from about 0.05% to about 2%, more preferably from about 0.07% to about 1% by weight of the total composition of an amphiphilic polymer selected from the groups consisting of amphiphilic alkoxylated polyalkyleneimine and mixtures thereof, preferably an amphiphilic alkoxylated polyalkyleneimine.

A preferred polyethyleneimine has the general structure of Formula (II):

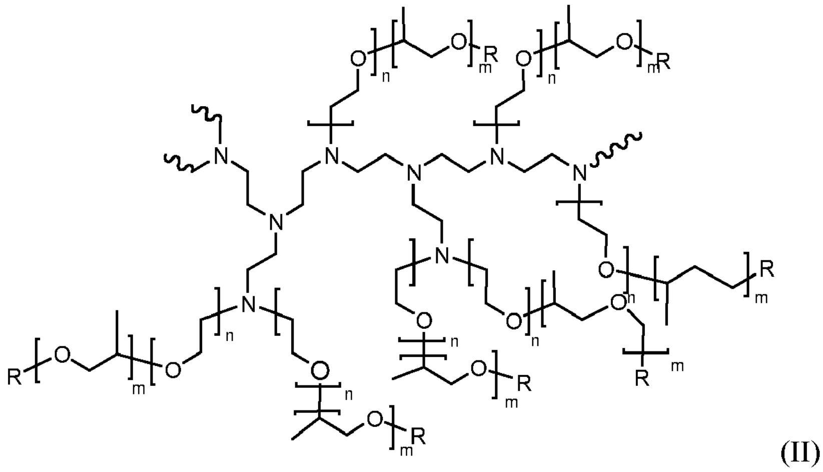

(II)

wherein the polyethyleneimine backbone has a weight average molecular weight of about 600 Da, n of Formula (II) has an average of about 24, m of Formula (II) has an average of about 16 and R of Formula (II) is selected from hydrogen, a $C_1$-$C_4$ alkyl and mixtures thereof, preferably hydrogen. The degree of permanent quaternization of Formula (II) may be from 0% to about 22% of the polyethyleneimine backbone nitrogen atoms, preferably 0%. The molecular weight of this polyethyleneimine preferably is between 25,000 and 30,000 most preferably about 28,000 Da.

Chelant

The detergent composition herein can comprise a chelant at a level of from 0.1% to 20%, preferably from 0.2% to 5%, more preferably from 0.2% to 3% by weight of total composition.

Preferably, the composition of the present invention comprises one or more chelants, preferably selected from the group comprising carboxylate chelants, amino carboxylate chelants, amino phosphonate chelants, and mixtures thereof. Preferably the chelants are selected from the group consisting of MGDA (methylglycine-N,N-diacetic acid), GLDA (glutamic-N,N- diacetic acid), and mixtures thereof.

Adjunct Ingredients

The detergent composition herein may optionally comprise a number of other adjunct ingredients such as builders (*e.g.*, preferably citrate), cleaning solvents, cleaning amines, conditioning polymers, cleaning polymers, surface modifying polymers, soil flocculating polymers, structurants, emollients, humectants, skin rejuvenating actives, enzymes, carboxylic acids, scrubbing particles, bleach and bleach activators, perfumes, malodor control agents, pigments, dyes, opacifiers, beads, pearlescent particles, microcapsules, inorganic cations such as alkaline earth metals such as Ca/Mg-ions, antibacterial agents, preservatives, viscosity adjusters (*e.g.*, salt such as NaCl, and other mono-, di- and trivalent salts) and pH adjusters and buffering means (*e.g.*, carboxylic acids such as citric acid, HCl, NaOH, KOH, alkanolamines, phosphoric and sulfonic acids, carbonates such as sodium carbonates, bicarbonates, sesquicarbonates, borates, silicates, phosphates, imidazole and alike).

Method of Washing

The present invention includes a method of manually washing soiled articles, preferably dishware, comprising the step of: delivering a composition of the invention into a volume of water to form a wash solution and immersing the soiled articles in the wash solution, wherein the soil on the soiled articles comprises at least one fatty acid selected from the group consisting of: saturated fatty acids, mono unsaturated fatty acids, di unsaturated fatty acids, tri unsaturated fatty acids, tetra unsaturated fatty acids, penta unsaturated fatty acids, hexa unsaturated fatty acids, and mixtures thereof. Preferred unsaturated fatty acids include: myristoleic acid, palmitoleic acid, sapienic acid, oleic acid, elaidic acid, vaccenic acid, linoleic acid, linoelaidic acid, $\alpha$-linolenic acid, $\gamma$-linolenic acid, gadoleic acid, $\alpha$-eleostearic acid, $\beta$-eleostearic acid, ricinoleic acid, eicosenic acid, arachidonic acid, eicosapentaenoic acid, erucic acid, docosadienoic acid, docosahexaenoic acid, tetracosenoic acid, and mixtures thereof, more preferably palmitoleic acid, oleic acid, linoleic acid, $\alpha$-linolenic acid, $\gamma$-linolenic acid, and mixtures thereof, more preferably oleic acid. Preferred saturated fatty acids include: myristic acid, palmitic acid, stearic acid, and mixtures thereof; more preferably, palmitic acid, stearic acid, and mixtures thereof.

Preferably the fatty acid alpha-dioxygenase according to the invention is present at a concentration of from

0.005 ppm to 15 ppm, preferably from 0.01ppm to 5 ppm, more preferably from 0.02 ppm to 0.5 ppm, in an aqueous wash liquor during the washing process. As such, the composition herein will be applied in its diluted form to the dishware. Soiled surfaces e.g. dishes are contacted with an effective amount, typically from 0.5 mL to 20 mL (per 25 dishes being treated), preferably from 3mL to 10 mL, of the detergent composition of the present invention, preferably in liquid form, diluted in water. The actual amount of detergent composition used will be based on the judgment of user, and will typically depend upon factors such as the particular product formulation of the composition, including the concentration of active ingredients in the composition, the number of soiled dishes to be cleaned, the degree of soiling on the dishes, and the like. Generally, from 0.01 mL to 150 mL, preferably from 3 mL to 40 mL of a liquid detergent composition of the invention is combined with from 2,000 mL to 20,000 mL, more typically from 5,000 mL to 15,000 mL of water in a sink having a volumetric capacity in the range of from 1,000 mL to 20,000 mL, more typically from 5,000 mL to 15,000 mL. The soiled dishes are immersed in the sink containing the diluted compositions then obtained, where contacting the soiled surface of the dish with a cloth, sponge, or similar article cleans them. The cloth, sponge, or similar article may be immersed in the detergent composition and water mixture prior to being contacted with the dish surface, and is typically contacted with the dish surface for a period of time ranged from 1 to 10 seconds, although the actual time will vary with each application and user. The contacting of cloth, sponge, or similar article to the surface is preferably accompanied by a concurrent scrubbing of the surface.

Alternatively, the composition of the present invention can be delivered directly onto the dishware or by contacting a cleaning implement (such as a sponge) comprising the composition with the dishware, before cleaning the dishware with the composition in the presence in water, and optionally, rinsing. Such direct application dishwashing methods are particularly beneficial for cleaning greasy dishware, and especially where the grease has been baked on.

The present invention includes the use one or more alpha-dioxygenase as described herein to provide increased suds longevity in an aqueous wash liquor comprising soil, wherein the soil comprises fatty acid. The enzymes are preferably comprised in a detergent composition, especially a detergent composition of the present invention, which is used for manually washing dishes.

## EXAMPLES

The following examples are provided to further illustrate the present invention.

### Example 1 - Sequence Similarity Network Analysis

Sequence similarity networks (SSN) are multidimensional versions of the more traditional one-dimensional BLAST analysis. In SSN, pairwise sequence relationships among different proteins are visualized. Each protein is illustrated as a "node", while each node is connected to other nodes by "edges". Only nodes representing proteins that are similar enough, based on amino acid sequence identity, are connected by edges. Thus, groups of highly similar proteins form clusters in an SSN diagram.

An SSN for alpha-dioxygenases was created using the EFI web tools (https://efi.igb.illinois.edu/. Rémi Zallot, Nils Oberg, and John A. Gerlt, The EFI Web Resource for Genomic Enzymology Tools: Leveraging Protein, Genome, and Metagenome Databases to Discover Novel Enzymes and Metabolic Pathways. Biochemistry 2019 58 (41), 4169-4182. https://doi.org/10.1021/acs.biochem.9b00735). Proteins with more than about 70% sequence identity were grouped together in clusters (see FIG. 1). Representative sequences of different clusters were selected for enzyme expression and kinetic characterization (see TABLE 2). Enzymes that convert multiple or single fatty acids at a higher rate (i.e. higher TON) are preferred in the current application.

SSNs have been used to identify and describe isofunctional families within enzyme families, e.g. clusters with different substrate specificity, providing an overview of sequence-function space (John A. Gerlt, Genomic enzymology: Web tools for leveraging protein family sequence-function space and genome context to discover novel functions, Biochemistry. Volume 56, 2017, Pages 4293-4308. https://doi.org/10.1021/acs.biochem.7b00614). It stands within reason that enzyme candidates within the same cluster can have similar properties due to the high level of sequence similarity. Thus, in addition to the preferred enzymes, other alpha-dioxygenases within their corresponding clusters are included as part of the current invention. For instance, alpha-dioxygenases with SEQ ID NO 15, 16, 17, 18, 19, 20, 21, 22, 23, 24, 25, 26, 28, 29, 30, 31, 32, 33, 34, 35, 36, 37, 38, 39, 40, 41, 42, 44, 46, 47, 48, 49, 50, 51, 52, 53, 54, 55, 56, 57, and 58 are very similar to SEQ ID NO 1, as their nodes are connected by edges in cluster 5 of the SSN (see FIG.1) and are therefore included as part of the current invention. Similarly, alpha-dioxygenases with SEQ ID NO 59, 60, 61, 62, 63, 64, 65, 66, 67, 68, 69, and 70 are very similar to SEQ ID NO 2, as their nodes are connected by edges in cluster 14 of the SSN, and are also included as part of the current invention. Alpha-dioxygenases with SEQ ID NO 71, 72, 73, 74, 75, 76, 77, 78, 79, 80, 82, 83, 84, 85, 86, 87, 89, 90, 91, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 153, 154, 155, 156,

157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, and 217 are very similar to SEQ ID NO: 3, 4, 5, and 6, as their nodes are connected by edges in cluster 1 of the SSN. Finally, alpha-dioxygenases with SEQ ID NO 218, 219, 220, 221, 222, 223, 224, 225, 226, 227, 228, 229, 230, 231, 232, 233, 234, 235, 236, 237, 238, 239, 240, 241, 242, 243, 244, 245, 246, and 247 are very similar to SEQ ID NO: 7, as their nodes are connected by edges in cluster 7 of the SSN.

Sequence alignment of fatty acid alpha-dioxygenases from clusters 5, 14, and 7 allowed calculating consensus sequences SEQ ID NO 276, 277, and 278, for each cluster, respectively.

Example 2 - Production of *Oryza sativa* αDOX

*Oryza sativa* αDOX (SEQ ID NO: 1) is a fatty acid alpha-dioxygenase that converts fatty acids into the corresponding 2-hydroperoxy fatty acids and is included as an example of the present invention. A codon optimized gene (SEQ ID NO: 248) encoding for an alpha-dioxygenase variant (SEQ ID NO: 249), including an N-terminal amino acid sequence containing a His-tag was designed and synthesized by Genscript. After gene synthesis, the protein was expressed and purified. In brief, the complete synthetic gene sequence was subcloned into a pET30a vector using the NdeI/HindIII cloning sites. For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing Magic Media (Thermo Fisher, Catalog # K6803) supplemented with kanamycin (50 mg/L) and incubated at 16 °C for 72 h. At an OD600nm = 0.5 - 1.0, 5-aminolevulinic acid (final concentration 0.5 mM) was added. Cells were harvested by centrifugation at 5000 rpm and 4°C and the pellet was lysed using a bacterial cell lysis buffer (B-PER - Themo Fisher, Waltham, MA). After centrifugation, the supernatant was collected, and the protein was purified by one-step purification using HisPur™ Ni-NTA Spin Columns (Thermo Scientific, Catalog # 88226) and standard protocols known in the art. The protein was concentrated using a 10 kDa MW cutoff Amicon Ultra centrifugal filter unit (Millipore Sigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 3 - Production of *Phaeosphaeria nodorum* αDOX

*Phaeosphaeria nodorum* αDOX (SEQ ID NO: 2) is a fatty acid alpha-dioxygenase that converts fatty acids into the corresponding 2-hydroperoxy fatty acids and is included as an example of the present invention. A codon optimized gene (SEQ ID NO: 250) encoding for an alpha-dioxygenase variant (SEQ ID NO: 251), including an N-terminal amino acid sequence containing a His-tag was designed and synthesized by Genscript. After gene synthesis, the protein was expressed and purified. In brief, the complete synthetic gene sequence was subcloned into a pET30a vector using the NdeI/HindIII cloning sites. For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing Magic Media (Thermo Fisher, Catalog # K6803) supplemented with kanamycin (50 mg/L) and incubated at 16 °C for 72 h. At an OD600nm = 0.5 - 1.0, 5-aminolevulinic acid (final concentration 0.5 mM) was added. Cells were harvested by centrifugation at 5000 rpm and 4°C and the pellet was lysed using a bacterial cell lysis buffer (B-PER - Themo Fisher, Waltham, MA). After centrifugation, the supernatant was collected, and the protein was purified by one-step purification using HisPur™ Ni-NTA Spin Columns (Thermo Scientific, Catalog # 88226) and standard protocols known in the art. The protein was concentrated using a 10 kDa MW cutoff Amicon Ultra centrifugal filter unit (Millipore Sigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 4 - Production of *Arabidopsis thaliana* αDOX

*Arabidopsis thaliana* αDOX (SEQ ID NO: 3) is a fatty acid alpha-dioxygenase that converts fatty acids into the corresponding 2-hydroperoxy fatty acids and is included as an example of the present invention. A codon optimized gene (SEQ ID NO: 252) encoding for an alpha-dioxygenase variant was designed and synthesized by Genscript. After synthesis, the gene was cloned into a modified version of pET28a, such as the final plasmid encoded for a αDOX variant including an N-terminal amino acid sequence containing a His-tag, an MBP tag, and a TEV protease cleavage site (SEQ ID NO: 253). For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing TB Media supplemented with kanamycin (50 mg/L) and incubated at 37 °C. At an OD600nm about 4, IPTG (final concentration 0.1 mM) and 5-aminolevulinic acid (final concentration 0.5 mM) were added

to the culture and further incubated at 16 °C for 16 h. Cells were harvested by centrifugation and the pellet was lysed. After centrifugation, the supernatant was collected, and the protein was purified by one-step purification using Ni-NTA affinity chromatography and standard protocols known in the art. The protein was stored in 1×PBS buffer containing 10% Glycerol and at pH 7.4. The purified enzyme was stored at -80 °C until use.

Example 5 - Production of *Pisum sativum* αDOX

*Pisum sativum* αDOX (SEQ ID NO: 4) is a fatty acid alpha-dioxygenase that converts fatty acids into the corresponding 2-hydroperoxy fatty acids and is included as an example of the present invention. A codon optimized gene (SEQ ID NO: 254) encoding for an alpha-dioxygenase variant (SEQ ID NO: 255), including an N-terminal amino acid sequence containing a His-tag was designed and synthesized by Genscript. After gene synthesis, the protein was expressed and purified. In brief, the complete synthetic gene sequence was subcloned into a pET30a vector using the NdeI/HindIII cloning sites. For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing Magic Media (Thermo Fisher, Catalog # K6803) supplemented with kanamycin (50 mg/L) and incubated at 16 °C for 72 h. At an OD600nm = 0.5 - 1.0, 5-aminolevulinic acid (final concentration 0.5 mM) was added. Cells were harvested by centrifugation at 5000 rpm and 4°C and the pellet was lysed using a bacterial cell lysis buffer (B-PER - Themo Fisher, Waltham, MA). After centrifugation, the supernatant was collected, and the protein was purified by one-step purification using HisPur™ Ni-NTA Spin Columns (Thermo Scientific, Catalog # 88226) and standard protocols known in the art. The protein was concentrated using a 10 kDa MW cutoff Amicon Ultra centrifugal filter unit (Millipore Sigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 6 - Production of *Gossypium raimondii* αDOX

*Gossypium raimondii* αDOX (SEQ ID NO: 5) is a fatty acid alpha-dioxygenase that converts fatty acids into the corresponding 2-hydroperoxy fatty acids and is included as an example of the present invention. A codon optimized gene (SEQ ID NO: 256) encoding for an alpha-dioxygenase variant (SEQ ID NO: 257), including an N-terminal amino acid sequence containing a His-tag was designed and synthesized by Genscript. After gene synthesis, the protein was expressed and purified. In brief, the complete synthetic gene sequence was subcloned into a pET30a vector using the NdeI/HindIII cloning sites. For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing Magic Media (Thermo Fisher, Catalog # K6803) supplemented with kanamycin (50 mg/L) and incubated at 16 °C for 72 h. At an OD600nm = 0.5 - 1.0, 5-aminolevulinic acid (final concentration 0.5 mM) was added. Cells were harvested by centrifugation at 5000 rpm and 4°C and the pellet was lysed using a bacterial cell lysis buffer (B-PER - Themo Fisher, Waltham, MA). After centrifugation, the supernatant was collected, and the protein was purified by one-step purification using HisPur™ Ni-NTA Spin Columns (Thermo Scientific, Catalog # 88226) and standard protocols known in the art. The protein was concentrated using a 10 kDa MW cutoff Amicon Ultra centrifugal filter unit (Millipore Sigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 7 - Production of *Nicotiana tabacum* αDOX

*Nicotiana tabacum* αDOX (SEQ ID NO: 6) is a fatty acid alpha-dioxygenase that converts fatty acids into the corresponding 2-hydroperoxy fatty acids and is included as an example of the present invention. A codon optimized gene (SEQ ID NO: 258) encoding for an alpha-dioxygenase variant (SEQ ID NO: 259), including an N-terminal amino acid sequence containing a His-tag was designed and synthesized by Genscript. After gene synthesis, the protein was expressed and purified. In brief, the complete synthetic gene sequence was subcloned into a pET30a vector using the NdeI/HindIII cloning sites. For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing Magic Media (Thermo Fisher, Catalog # K6803) supplemented with kanamycin (50 mg/L) and incubated at 16 °C for 72 h. At an OD600nm = 0.5 - 1.0, 5-aminolevulinic acid (final concentration 0.5 mM) was added. Cells were harvested by centrifugation at 5000 rpm and 4°C and the pellet was lysed using a bacterial cell lysis buffer (B-PER - Themo Fisher, Waltham, MA). After centrifugation, the supernatant was collected, and the protein was purified by one-step purification using HisPur™ Ni-NTA Spin Columns (Thermo Scientific, Catalog # 88226) and standard protocols known in the art. The protein was concentrated using a 10 kDa MW cutoff Amicon Ultra

centrifugal filter unit (Millipore Sigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 8 - Production of *Mycolicibacterium fortuitum* αDOX

*Mycolicibacterium fortuitum* αDOX (SEQ ID NO: 7) is a fatty acid alpha-dioxygenase that converts fatty acids into the corresponding 2-hydroperoxy fatty acids and is included as an example of the present invention. A codon optimized gene (SEQ ID NO: 260) encoding for an alpha-dioxygenase variant (SEQ ID NO: 261), including an N-terminal amino acid sequence containing a His-tag was designed and synthesized by Genscript. After gene synthesis, the protein was expressed and purified. In brief, the complete synthetic gene sequence was subcloned into a pET30a vector using the NdeI/HindIII cloning sites. For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing Magic Media (Thermo Fisher, Catalog # K6803) supplemented with kanamycin (50 mg/L) and incubated at 16 °C for 72 h. At an OD600nm = 0.5 - 1.0, 5-aminolevulinic acid (final concentration 0.5 mM) was added. Cells were harvested by centrifugation at 5000 rpm and 4°C and the pellet was lysed using a bacterial cell lysis buffer (B-PER - Themo Fisher, Waltham, MA). After centrifugation, the supernatant was collected, and the protein was purified by one-step purification using HisPur™ Ni-NTA Spin Columns (Thermo Scientific, Catalog # 88226) and standard protocols known in the art. The protein was concentrated using a 10 kDa MW cutoff Amicon Ultra centrifugal filter unit (Millipore Sigma, Catalog# UFC901024), followed by desalting using a disposable PD-10 50 mM Tris-HCl, 500 mM NaCl, and 10% Glycerol at pH 8.0. The purified enzyme was stored at -80 °C until use.

Example 9 (Comparative) - Production of *Solanum lycopersicum* αDOX

*Solanum lycopersicum* αDOX (SEQ ID NO: 8) is a fatty acid alpha-dioxygenase that converts fatty acids into the corresponding 2-hydroperoxy fatty acids and is included as a comparative example of the present invention. A codon optimized gene (SEQ ID NO: 262) encoding for an alpha-dioxygenase variant (SEQ ID NO: 263), including an N-terminal amino acid sequence containing a His-tag was designed and synthesized by Genscript. After gene synthesis, the protein was expressed and purified. In brief, the complete synthetic gene sequence was subcloned into a pET30a vector using the NdeI/HindIII cloning sites. For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing Magic Media (Thermo Fisher, Catalog # K6803) supplemented with kanamycin (50 mg/L) and incubated at 16 °C for 72 h. At an OD600nm = 0.5 - 1.0, 5-aminolevulinic acid (final concentration 0.5 mM) was added. Cells were harvested by centrifugation at 5000 rpm and 4°C and the pellet was lysed using a bacterial cell lysis buffer (B-PER - Themo Fisher, Waltham, MA). After centrifugation, the supernatant was collected, and the protein was purified by one-step purification using HisPur™ Ni-NTA Spin Columns (Thermo Scientific, Catalog # 88226) and standard protocols known in the art. The protein was concentrated using a 10 kDa MW cutoff Amicon Ultra centrifugal filter unit (Millipore Sigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 10 (Comparative) - Production of *Medicago truncatula* αDOX

*Medicago truncatula* αDOX (SEQ ID NO: 9) is a fatty acid alpha-dioxygenase that converts fatty acids into the corresponding 2-hydroperoxy fatty acids and is included as a comparative example of the present invention. A codon optimized gene (SEQ ID NO: 264) encoding for an alpha-dioxygenase variant (SEQ ID NO: 265), including an N-terminal amino acid sequence containing a His-tag was designed and synthesized by Genscript. After gene synthesis, the protein was expressed and purified. In brief, the complete synthetic gene sequence was subcloned into a pET30a vector using the NdeI/HindIII cloning sites. For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing Magic Media (Thermo Fisher, Catalog # K6803) supplemented with kanamycin (50 mg/L) and incubated at 16 °C for 72 h. At an OD600nm = 0.5 - 1.0, 5-aminolevulinic acid (final concentration 0.5 mM) was added. Cells were harvested by centrifugation at 5000 rpm and 4°C and the pellet was lysed using a bacterial cell lysis buffer (B-PER - Themo Fisher, Waltham, MA). After centrifugation, the supernatant was collected, and the protein was purified by one-step purification using HisPur™ Ni-NTA Spin Columns (Thermo Scientific, Catalog # 88226) and standard protocols known in the art. The protein was concentrated using a 10 kDa MW cutoff Amicon Ultra centrifugal filter unit (Millipore Sigma, Catalog# UFC901024), followed by desalting using a disposable PD-10 desalting column (GE Healthcare Life Sciences, Catalog# 17085101) and a buffer containing 50 mM Tris-HCl,

500 mM NaCl, and 10% Glycerol at pH 8.0. The purified enzyme was stored at -80 °C until use.

Example 11 (Comparative) - Production of *Arabidopsis thaliana* αDOX

*Arabidopsis thaliana* αDOX (SEQ ID NO: 10) is a fatty acid alpha-dioxygenase that converts fatty acids into the corresponding 2-hydroperoxy fatty acids and is included as a comparative example of the present invention. A codon optimized gene (SEQ ID NO: 266) encoding for an alpha-dioxygenase variant was designed and synthesized by Genscript. After synthesis, the gene was cloned into a modified version of pET28a, such as the final plasmid encoded for a αDOX variant including an N-terminal amino acid sequence containing a His-tag, an MBP tag, and a TEV protease cleavage site (SEQ ID NO: 267). For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing TB Media supplemented with kanamycin (50 mg/L) and incubated at 37 °C. At an OD600nm about 3.8, IPTG (final concentration 0.1 mM) and 5-aminolevulinic acid (final concentration 0.25 mM) were added to the culture and further incubated at 16 °C for 16 h. Cells were harvested by centrifugation and the pellet was lysed. After centrifugation, the supernatant was collected, and the protein was purified by one-step purification using Ni-NTA affinity chromatography and standard protocols known in the art. The protein was stored in 1×PBS buffer containing 10% Glycerol and at pH 7.4. The purified enzyme was stored at -80 °C until use.

Example 12 (Comparative)- Production of *Citrus sinensis* αDOX

*Citrus sinensis* αDOX (SEQ ID NO: 11) is a fatty acid alpha-dioxygenase that converts fatty acids into the corresponding 2-hydroperoxy fatty acids and is included as a comparative example of the present invention. A codon optimized gene (SEQ ID NO: 268) encoding for an alpha-dioxygenase variant (SEQ ID NO: 269), including an N-terminal amino acid sequence containing a His-tag was designed and synthesized by Genscript. After gene synthesis, the protein was expressed and purified. In brief, the complete synthetic gene sequence was subcloned into a pET30a vector using the NdeI/HindIII cloning sites. For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing Magic Media (Thermo Fisher, Catalog # K6803) supplemented with kanamycin (50 mg/L) and incubated at 16 °C for 72 h. At an OD600nm = 0.5 - 1.0, 5-aminolevulinic acid (final concentration 0.5 mM) was added. Cells were harvested by centrifugation at 5000 rpm and 4°C and the pellet was lysed using a bacterial cell lysis buffer (B-PER - Themo Fisher, Waltham, MA). After centrifugation, the supernatant was collected, and the protein was purified by one-step purification using HisPur™ Ni-NTA Spin Columns (Thermo Scientific, Catalog # 88226) and standard protocols known in the art. The protein was concentrated using a 10 kDa MW cutoff Amicon Ultra centrifugal filter unit (Millipore Sigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 13 (Comparative)- Production of *Pseudomonas alcaligenes* αDOX

*Pseudomonas alcaligenes* αDOX (SEQ ID NO: 12) is a fatty acid alpha-dioxygenase that converts fatty acids into the corresponding 2-hydroperoxy fatty acids and is included as a comparative example of the present invention. A codon optimized gene (SEQ ID NO: 270) encoding for an alpha-dioxygenase variant (SEQ ID NO: 271), including an N-terminal amino acid sequence containing a His-tag was designed and synthesized by Genscript. After gene synthesis, the protein was expressed and purified. In brief, the complete synthetic gene sequence was subcloned into a pET30a vector using the NdeI/HindIII cloning sites. For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing Magic Media (Thermo Fisher, Catalog # K6803) supplemented with kanamycin (50 mg/L) and incubated at 16 °C for 72 h. At an OD600nm = 0.5 - 1.0, 5-aminolevulinic acid (final concentration 0.5 mM) was added. Cells were harvested by centrifugation at 5000 rpm and 4°C and the pellet was lysed using a bacterial cell lysis buffer (B-PER - Themo Fisher, Waltham, MA). After centrifugation, the supernatant was collected, and the protein was purified by one-step purification using HisPur™ Ni-NTA Spin Columns (Thermo Scientific, Catalog # 88226) and standard protocols known in the art. The protein was concentrated using a 10 kDa MW cutoff Amicon Ultra centrifugal filter unit (Millipore Sigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 14 (Comparative)- Production of *Streptomyces avermitilis* αDOX

*Streptomyces avermitilis* αDOX (SEQ ID NO: 13) is a fatty acid alpha-dioxygenase that converts fatty acids into the corresponding 2-hydroperoxy fatty acids and is included as a comparative example of the present invention. A codon optimized gene (SEQ ID NO: 272) encoding for an alpha-dioxygenase variant (SEQ ID NO: 273), including an N-terminal amino acid sequence containing a His-tag was designed and synthesized by Genscript. After gene synthesis, the protein was expressed and purified. In brief, the complete synthetic gene sequence was subcloned into a pET30a vector using the NdeI/HindIII cloning sites. For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing Magic Media (Thermo Fisher, Catalog # K6803) supplemented with kanamycin (50 mg/L) and incubated at 16 °C for 72 h. At an OD600nm = 0.5 - 1.0, 5-aminolevulinic acid (final concentration 0.5 mM) was added. Cells were harvested by centrifugation at 5000 rpm and 4°C and the pellet was lysed using a bacterial cell lysis buffer (B-PER - Themo Fisher, Waltham, MA). After centrifugation, the supernatant was collected, and the protein was purified by one-step purification using HisPur™ Ni-NTA Spin Columns (Thermo Scientific, Catalog # 88226) and standard protocols known in the art. The protein was concentrated using a 10 kDa MW cutoff Amicon Ultra centrifugal filter unit (Millipore Sigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 15 (Comparative)- Production *of Mycolicibacterium fortuitum* αDOX

*Mycolicibacterium fortuitum* αDOX (SEQ ID NO: 14) is a fatty acid alpha-dioxygenase that converts fatty acids into the corresponding 2-hydroperoxy fatty acids and is included as a comparative example of the present invention. A codon optimized gene (SEQ ID NO: 274) encoding for an alpha-dioxygenase variant (SEQ ID NO: 275), including an N-terminal amino acid sequence containing a His-tag was designed and synthesized by Genscript. After gene synthesis, the protein was expressed and purified. In brief, the complete synthetic gene sequence was subcloned into a pET30a vector using the NdeI/HindIII cloning sites. For heterologous expression, *Escherichia coli* BL21 (DE3) cells were transformed with the recombinant plasmid and a single colony was inoculated into LB medium containing kanamycin (50 mg/L). A pre-starter culture was then inoculated into flasks containing Magic Media (Thermo Fisher, Catalog # K6803) supplemented with kanamycin (50 mg/L) and incubated at 16 °C for 72 h. At an OD600nm = 0.5 - 1.0, 5-aminolevulinic acid (final concentration 0.5 mM) was added. Cells were harvested by centrifugation at 5000 rpm and 4°C and the pellet was lysed using a bacterial cell lysis buffer (B-PER - Themo Fisher, Waltham, MA). After centrifugation, the supernatant was collected, and the protein was purified by one-step purification using HisPur™ Ni-NTA Spin Columns (Thermo Scientific, Catalog # 88226) and standard protocols known in the art. The protein was concentrated using a 10 kDa MW cutoff Amicon Ultra centrifugal filter unit (Millipore Sigma, Catalog# UFC901024), followed by desalting using a disposable PD-10 desalting column (GE Healthcare Life Sciences, Catalog# 17085101) and a buffer containing 50 mM Tris-HCl, 500 mM NaCl, and 10% Glycerol at pH 8.0. The purified enzyme was stored at -80 °C until use.

Example 16 - Enzyme activity assays

Reactions with alpha-dioxygenases were performed as follows. An aliquot of fatty acid in ethanol (final concentration 100 μM) was resuspended in buffer (50 mM HEPES at pH 9.0) in a scintillation vial. The reaction was started by addition of enzyme (final concentration 6 ppm). The reaction was incubated for up to 10 minutes at 37°C with 200 rpm agitation and the conversion was monitored using an oxygen sensor. The TON (in s-1) were calculated as the ratio between the initial rate of oxygen consumption (in μM/s) and the concentration of enzyme (in μM). The results are summarized in TABLE 2. Aliquots of 100 μL of the reaction solutions were collected and mixed with 900 μL of isopropyl alcohol to stop the reactions. Analysis of the samples was performed by reversed-phase LC/MS/MS to confirm the conversion of the fatty acids.

TABLE 2. TON of Fatty Acids by Different Alpha-Dioxygenases.

| Example | SEQ ID | SSN Cluster | Accession Number | Palmitic Acid, TON [$s^{-1}$] | Stearic Acid, TON [$s^{-1}$] | Oleic Acid, TON [$s^{-1}$] |
|---|---|---|---|---|---|---|
| 2 | 1 | 5 | Q9M5J1 | 20.58 | 8.02 | 5.54 |
| 3 | 2 | 14 | Q0URU2 | 20.63 | 8.80 | 0.99 |
| 4 | 3 | 1 | Q9SGH6 | 1.01 | 0.47 | 0.14 |

(continued)

| Example | SEQ ID | SSN Cluster | Accession Number | Palmitic Acid, TON [$s^{-1}$] | Stearic Acid, TON [$s^{-1}$] | Oleic Acid, TON [$s^{-1}$] |
|---|---|---|---|---|---|---|
| 5 | 4 | 1 | Q5GQ66 | 1.92 | 0.80 | 1.40 |
| 6 | 5 | 1 | A0A0D2W0W0 | 2.56 | 1.68 | 1.68 |
| 7 | 6 | 1 | 082031 | 3.07 | 2.66 | 0.78 |
| 8 | 7 | 7 | K0VMX8 | 0.00 | 1.66 | 0.00 |
| 9* | 8 | 3 | Q5WM33 | 0.00 | 0.12 | 0.00 |
| 10* | 9 | 3 | A4PRI0 | 0.57 | 0.02 | 0.06 |
| 11* | 10 | 3 | Q9C9U3 | 0.54 | 0.13 | 0.13 |
| 12* | 11 | 3 | A0A067FHE4 | 0.29 | 0.00 | 0.00 |
| 13* | 12 | 15 | U3BA19 | 0.36 | 0.21 | 0.00 |
| 14* | 13 | 16 | Q82M86 | 0.00 | 0.10 | 0.00 |
| 15* | 14 | 8 | K0VM18 | 0.21 | 0.03 | 0.00 |
| *: Comparative examples | | | | | | |

Example 17 - Exemplary Manual Dish-Washing Detergent Compositions

Manual dish-washing detergent compositions comprising fatty acids alpha-dioxygenases (SEQ ID NO: 1 or 2) according to the invention are shown in TABLE 3. The enzymes can be produced following the protocols described in Examples 2 and 3 or similar procedures described in the art.

TABLE 3: Detergent Compositions

| Ingredient | Wt% | Wt% |
|---|---|---|
| Sodium alkyl ethoxy sulfate (C1213EO0.6S) | 22.91% | 22.91% |
| n-C12-14 Di Methyl Amine Oxide | 7.64% | 7.64% |
| Lutensol® XP80 (non-ionic surfactant supplied by BASF) | 0.45% | 0.45% |
| Sodium Chloride | 1.2% | 1.2% |
| Poly Propylene Glycol (MW 2000) | 1% | 1% |
| Ethanol | 2% | 2% |
| Sodium Hydroxide | 0.24% | 0.24% |
| *Oryza sativa* αDOX (SEQ ID NO: 1) | 0.1% | 0.0% |
| *Phaeosphaeria nodorum* αDOX (SEQ ID NO: 2) | 0.0% | 0.1% |
| Minors (perfume, preservative, dye) + water | To 100 % | To 100 % |
| pH (@ 10% solution) | 9 | 9 |

All percentages and ratios given for enzymes are based on active protein. All percentages and ratios herein are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A detergent composition comprising:

   a) a fatty acid alpha-dioxygenase; wherein said fatty acid alpha-dioxygenase comprises a polypeptide sequence having at least 65% identity to one or more sequences selected from the group consisting of: SEQ ID NO: 1, 2, 3, 4, 5, 6, 7, and functional fragments thereof; and
   b) a surfactant system.

2. The composition according to claim 1, wherein said fatty acid alpha-dioxygenase comprises a polypeptide sequence having at least 70%, preferably at least 80%, preferably at least 90%, preferably at least 95%, preferably at least 98% or preferably even 100% identity to one or more sequences selected from the group consisting of: SEQ ID NO: 1, 2, and their functional fragments; more preferably SEQ ID NO: 1.

3. The composition according to any preceding claims, wherein said fatty acid alpha-dioxygenase comprises a polypeptide sequence comprising the amino acids N145, (H/R)157, W213, D214, S216, Y219, G220, R230, G256, G264, H276, N277, A308, K309, H311, W315, F375, Y379, R380, H382, S435, G437, N448, Y520, G532, F549, F552, R559, and G579; and wherein said positions are numbered with reference to SEQ ID NO: 1.

4. The composition according to any preceding claims, wherein said fatty acid alpha-dioxygenase comprises a polypeptide sequence selected from the group consisting of: SEQ ID NO: 276, 277, and 278; preferably SEQ ID NO: 276, and 277; and most preferably SEQ ID NO: 276.

5. The composition according to any preceding claims, wherein said fatty acid alpha-dioxygenase comprises a polypeptide sequence having at least about 90%, 95%, 98%, 100% identity to one or more sequences selected from the group consisting of: SEQ ID NO: 1, 2, 3, 4, 5, 6, 7, 15, 16, 17, 18, 19, 20, 21, 22, 23, 24, 25, 26, 28, 29, 30, 31, 32, 33, 34, 35, 36, 37, 38, 39, 40, 41, 42, 44, 46, 47, 48, 49, 50, 51, 52, 53, 54, 55, 56, 57, 58, 71, 72, 73, 74, 75, 76, 77, 78, 79, 80, 82, 83, 84, 85, 86, 87, 89, 90, 91, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, and their functional fragments thereof; preferably SEQ ID NO: 1, 2, 15, 16, 17, 18, 19, 20, 21, 22, 23, 24, 25, 26, 28, 29, 30, 31, 32, 33, 34, 35, 36, 37, 38, 39, 40, 41, 42, 44, 46, 47, 48, 49, 50, 51, 52, 53, 54, 55, 56, 57, 58, 59, 60, 61, 62, 63, 64, 65, 66, 67, 68, 69, 70, and their functional fragments thereof; more preferably SEQ ID NO: 1, 15, 16, 17, 18, 19, 20, 21, 22, 23, 24, 25, 26, 28, 29, 30, 31, 32, 33, 34, 35, 36, 37, 38, 39, 40, 41, 42, 44, 46, 47, 48, 49, 50, 51, 52, 53, 54, 55, 56, 57, 58; most preferably SEQ ID NO: 1

6. The composition according to any preceding claims, wherein said one or more fatty acid alpha-dioxygenase are present in an amount of from 0.0001 wt% to 1 wt%, preferably from 0.001 wt% to 0.2 wt%, by weight of the cleaning composition, based on active protein

7. The composition according to any preceding claims, further comprising one or more co-enzymes selected from the group consisting of: fatty-acid peroxidases (EC 1.11.1.3), unspecific peroxygenases (EC 1.11.2.1), plant seed peroxygenases (EC 1.11.2.3), fatty acid peroxygenases (EC1.11.2.4), linoleate diol synthases (EC 1.13.11.44), 5,8-linoleate diol synthases (EC 1.13.11.60 and EC 5.4.4.5), 7,8-linoleate diol synthases (EC 1.13.11.60 and EC 5.4.4.6), 9,14-linoleate diol synthases (EC 1.13.11.B1), 8,11-linoleate diol synthases, oleate diol synthases, other linoleate diol synthases, unspecific monooxygenase (EC 1.14.14.1), alkane 1-monooxygenase (EC 1.14.15.3), oleate 12-hydroxylases (EC 1.14.18.4), fatty acid amide hydrolases (EC 3.5.1.99), fatty acid photodecarboxylases (EC 4.1.1.106), oleate hydratases (EC 4.2.1.53), linoleate isomerases (EC 5.2.1.5), linoleate (10E,12Z)-isomerases (EC

5.3.3.B2), non-heme fatty acid decarboxylases, P450 fatty acid decarboxylases, amylases, lipases, proteases, cellulases, and mixtures thereof; preferably fatty-acid peroxidases (EC 1.11.1.3), unspecific peroxygenases (EC 1.11.2.1), plant seed peroxygenases (EC 1.11.2.3), and fatty acid peroxygenases (EC1.11.2.4), non-heme fatty acid decarboxylases, P450 fatty acid decarboxylases, and mixtures thereof.

**8.** The composition according to any preceding claims, wherein the surfactant system is present in an amount of from 1 wt% to 60 wt%, preferably from 5 wt% to 50 wt%, by weight of the composition.

**9.** The composition according to any preceding claims, wherein the surfactant system comprises one or more anionic surfactants and one or more co-surfactants, preferably the co-surfactants are selected from the group consisting of amphoteric surfactant, zwitterionic surfactant, and mixtures thereof, preferably the amphoteric surfactant is amine oxide surfactant and wherein the zwitterionic surfactant is betaine surfactant, preferably wherein the weight ratio of the anionic surfactants to the co-surfactants is less than 9:1, more preferably from 5:1 to 1:1, more preferably from 4:1 to 2:1.

**10.** The composition according to claim 9, wherein the anionic surfactants are a mixture of alkyl sulfates and alkyl alkoxy sulfates, the co-surfactants is alkyl dimethyl amine oxide, and wherein the weight ratio of the anionic surfactants to the co-surfactants is from 4:1 to 2:1.

**11.** The composition according to any preceding claims, further comprising an enzyme stabilizer selected from the group consisting of chemical and physical stabilizers, preferably wherein the enzyme is physical stabilized by encapsulating the enzyme.

**12.** The composition according to any preceding claims, further comprising a chelant, preferably selected from the group consisting of carboxylate chelant, amino carboxylate chelant, amino phosphonate chelant, and mixtures thereof, preferably the chelant is selected from the group consisting of MGDA (methylglycine-N,N-diacetic acid), GLDA (glutamic-N,N- diacetic acid), and mixtures thereof.

**13.** A method of manually washing dishware, comprising the step of: delivering a composition according to any preceding claims into a volume of water to form a wash solution and immersing the soiled articles in the wash solution, wherein the soil on the soiled articles comprises at least one fatty acid selected from the group consisting of: saturated fatty acids, mono unsaturated fatty acids, di unsaturated fatty acids, tri unsaturated fatty acids, tetra unsaturated fatty acids, penta unsaturated fatty acids, hexa unsaturated fatty acids, and mixtures thereof; preferably myristic acid, palmitic acid, stearic acid, myristoleic acid, palmitoleic acid, sapienic acid, oleic acid, elaidic acid, vaccenic acid, linoleic acid, linoelaidic acid, $\alpha$-linolenic acid, $\gamma$-linolenic acid, gadoleic acid, $\alpha$-eleostearic acid, $\beta$-eleostearic acid, ricinoleic acid, eicosenic acid, arachidonic acid, eicosapentaenoic acid, erucic acid, docosadienoic acid, docosahexaenoic acid, tetracosenoic acid, and mixtures thereof, preferably palmitic acid, stearic acid, oleic acid, linoleic acid, and mixtures thereof..

**14.** The method according to claim 13, wherein the fatty acid alpha-dioxygenase is present at a concentration of from 0.005 ppm to 15 ppm, preferably from 0.01ppm to 5 ppm, more preferably from 0.02 ppm to 0.5 ppm, based on active protein, in the wash solution during the washing step.

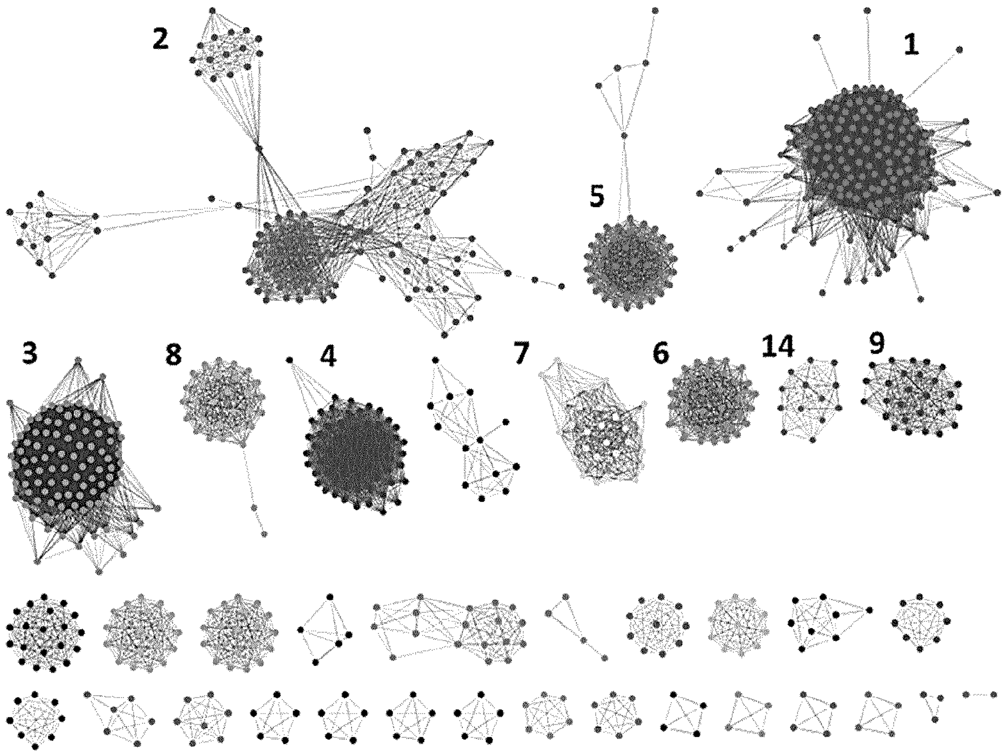


FIG. 1. Sequence similarity network of alpha-dioxygenases.

Europäisches Patentamt
European Patent Office
Office européen des brevets

# EUROPEAN SEARCH REPORT

Application Number
EP 20 20 0964

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>Y | EP 3 483 251 A1 (PROCTER & GAMBLE [US]) 15 May 2019 (2019-05-15)<br>* paragraphs [0006], [0007]; claims 1-15; examples 1-3; sequences 6, 23 * | 1-3,5-14<br>1-3,5-14 | INV.<br>C11D1/83<br>C11D1/94<br>C11D3/386<br>C11D11/00<br>C12N9/04<br>C12N9/02<br>C12N9/88 |
| A | US 2019/144794 A1 (VELASQUEZ JUAN ESTEBAN [US] ET AL) 16 May 2019 (2019-05-16)<br>* paragraphs [0003] - [0006]; claims 1-40 * | 1-3,5-14 | |
| A,D | EP 3 483 244 A1 (PROCTER & GAMBLE [US]) 15 May 2019 (2019-05-15)<br>* claims 1-15 * | 1-3,5-14 | |
| Y | DATABASE UniProt [Online]<br><br>5 September 2006 (2006-09-05), "SubName: Full=Uncharacterized protein {ECO:0000313¦EMBL:EAT86586.2};", XP002803173, retrieved from EBI accession no. UNIPROT:Q0URU2<br>Database accession no. Q0URU2<br>* the whole document * | 1-3,5-14 | TECHNICAL FIELDS SEARCHED (IPC)<br>C11D<br>C12N |

~~The present search report has been drawn up for all claims~~

3

| Place of search | Date of completion of the search | Examiner |
|---|---|---|
| The Hague | 2 June 2021 | van Klompenburg, Wim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 20 20 0964

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

1-14(partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 20 20 0964

The Search Division considers that the present European patent application does not comply with the requirements of unity of invention and relates to several inventions or groups of inventions, namely:

1. claims: 1-3, 5-14(all partially)

A detergent composition comprising a surfactant system and a fatty acid alpha dioxygenase having at least 65% sequence identity to SEQ ID NO:1. A method of manually washing dishware using the detergent composition.

---

2. claims: 1-3, 5-14(all partially)

A detergent composition comprising a surfactant system and a fatty acid alpha dioxygenase having at least 65% sequence identity to SEQ ID NO:2. A method of manually washing dishware using the detergent composition.

---

3. claims: 1-3, 5-14(all partially)

A detergent composition comprising a surfactant system and a fatty acid alpha dioxygenase having at least 65% sequence identity to SEQ ID NO:3. A method of manually washing dishware using the detergent composition.

---

4. claims: 1-3, 5-14(all partially)

A detergent composition comprising a surfactant system and a fatty acid alpha dioxygenase having at least 65% sequence identity to SEQ ID NO:4. A method of manually washing dishware using the detergent composition.

---

5. claims: 1-3, 5-14(all partially)

A detergent composition comprising a surfactant system and a fatty acid alpha dioxygenase having at least 65% sequence identity to SEQ ID NO:5. A method of manually washing dishware using the detergent composition.

---

6. claims: 1-3, 5-14(all partially)

A detergent composition comprising a surfactant system and a fatty acid alpha dioxygenase having at least 65% sequence identity to SEQ ID NO:6. A method of manually washing dishware using the detergent composition.

---

7. claims: 1-3, 5-14(all partially)

A detergent composition comprising a surfactant system and a

Europäisches Patentamt
European Patent Office
Office européen des brevets

# LACK OF UNITY OF INVENTION SHEET B

Application Number
EP 20 20 0964

The Search Division considers that the present European patent application does not comply with the requirements of unity of invention and relates to several inventions or groups of inventions, namely:

fatty acid alpha dioxygenase having at least 65% sequence identity to SEQ ID NO:7. A method of manually washing dishware using the detergent composition.

---

8. claims: 1-14(partially)

A detergent composition comprising a surfactant system and a fatty acid alpha dioxygenase according to SEQ ID NO:276. A method of manually washing dishware using the detergent composition.

---

9. claims: 1-14(partially)

A detergent composition comprising a surfactant system and a fatty acid alpha dioxygenase according to SEQ ID NO:277. A method of manually washing dishware using the detergent composition.

---

10. claims: 1-14(partially)

A detergent composition comprising a surfactant system and a fatty acid alpha dioxygenase according to SEQ ID NO:278. A method of manually washing dishware using the detergent composition.

---

11-213. claims: 1-3, 5-14(all partially)

A detergent composition comprising a surfactant system and a fatty acid alpha dioxygenase having at least 90% sequence identity to SEQ ID NO:15, 16, 17, 18, 19, 20, 21, 22, 23, 24, 25, 26, 28, 29, 30, 31, 32, 33, 34, 35, 36, 37, 38, 39, 40, 41, 42, 44, 46, 47, 48, 49, 50, 51, 52, 53, 54, 55, 56, 57, 58, 71, 72, 73, 74, 75, 76, 77, 78, 79, 80, 82, 83, 84, 85, 86, 87, 89, 90, 91, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247. Such that invention 11 is identified by SEQ ID 15,

# LACK OF UNITY OF INVENTION SHEET B

Application Number
EP 20 20 0964

The Search Division considers that the present European patent application does not comply with the requirements of unity of invention and relates to several inventions or groups of inventions, namely:

invention 12 by SEQ ID 16 etc.. A method of manually washing dishware using the detergent composition.

---

## ANNEX TO THE EUROPEAN SEARCH REPORT ON EUROPEAN PATENT APPLICATION NO. EP 20 20 0964

This annex lists the patent family members relating to the patent documents cited in the above-mentioned European search report. The members are as contained in the European Patent Office EDP file on
The European Patent Office is in no way liable for these particulars which are merely given for the purpose of information.

02-06-2021

| Patent document cited in search report | Publication date | Patent family member(s) | Publication date |
|---|---|---|---|
| EP 3483251 A1 | 15-05-2019 | CA 3079408 A1 | 16-05-2019 |
| | | CN 111263804 A | 09-06-2020 |
| | | EP 3483243 A1 | 15-05-2019 |
| | | EP 3483244 A1 | 15-05-2019 |
| | | EP 3483251 A1 | 15-05-2019 |
| | | JP 2021502453 A | 28-01-2021 |
| | | JP 2021502456 A | 28-01-2021 |
| | | US 2019144790 A1 | 16-05-2019 |
| | | US 2019144791 A1 | 16-05-2019 |
| | | WO 2019094531 A1 | 16-05-2019 |
| | | WO 2019094532 A1 | 16-05-2019 |
| | | WO 2019094534 A1 | 16-05-2019 |
| US 2019144794 A1 | 16-05-2019 | CN 111247234 A | 05-06-2020 |
| | | EP 3710568 A1 | 23-09-2020 |
| | | US 2019144794 A1 | 16-05-2019 |
| | | WO 2019094898 A1 | 16-05-2019 |
| EP 3483244 A1 | 15-05-2019 | CA 3079408 A1 | 16-05-2019 |
| | | CN 111263804 A | 09-06-2020 |
| | | EP 3483243 A1 | 15-05-2019 |
| | | EP 3483244 A1 | 15-05-2019 |
| | | EP 3483251 A1 | 15-05-2019 |
| | | JP 2021502453 A | 28-01-2021 |
| | | JP 2021502456 A | 28-01-2021 |
| | | US 2019144790 A1 | 16-05-2019 |
| | | US 2019144791 A1 | 16-05-2019 |
| | | WO 2019094531 A1 | 16-05-2019 |
| | | WO 2019094532 A1 | 16-05-2019 |
| | | WO 2019094534 A1 | 16-05-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2017321161 A1
- US 2003166485 A1
- EP 3483243 A1
- EP 3483251 A1
- EP 3483244 A1
- WO 201219844 A
- WO 201219849 A
- WO 201219848 A
- US 3915903 A


### Non-patent literature cited in the description


- Water Soluble Film Flakes Incorporating Functional Ingredients. IP.COM JOURNAL. IP.COM INC, 02 January 2014
- **NEEDLEMAN ; WUNSCH.** *J. Mol. Biol.,* 1970, vol. 48, 443-453
- **HENIKOFF S. ; HENIKOFF J.G.** *P.N.A.S. USA,* 1992, vol. 89, 10915-10919
- **PETERSEN TN ; BRUNAK S. ; VON HEIJNE G. ; NIELSEN H.** *Nature Methods,* 2011, vol. 8, 785-786
- **YI et al.** *Proc. Biochem.,* 2007, vol. 42, 895-898
- **MARTIN et al.** *Appl. Microbiol. Biotechnol.,* 2007, vol. 76, 843-851
- **KOSZELEWSKI et al.** *J. Mol. Cat. B: Enz.,* 2010, vol. 63, 39-44
- **TRUPPO et al.** *Org. Proc. Res. Develop.*
- **MATEO et al.** *Biotechnol. Prog.,* 2002, vol. 18, 629-34
- **RÉMI ZALLOT ; NILS OBERG ; JOHN A.** Gerlt, The EFI Web Resource for Genomic Enzymology Tools: Leveraging Protein, Genome, and Metagenome Databases to Discover Novel Enzymes and Metabolic Pathways. *Biochemistry,* 2019, vol. 58 (41), 4169-4182, https://efi.igb.illinois.edu
- **JOHN A. GERLT.** Genomic enzymology: Web tools for leveraging protein family sequence-function space and genome context to discover novel functions. *Biochemistry,* 2017, vol. 56, 4293-4308, https://doi.org/10.1021/acs.biochem.7b00614